(19)

Europäisches Patentamt
European Patent Office
Office européen des brevets

(11) **EP 2 811 016 B1**

(12) **EUROPEAN PATENT SPECIFICATION**

(45) Date of publication and mention of the grant of the patent:
**04.10.2017 Bulletin 2017/40**

(51) Int Cl.:
*C12N 5/073* (2010.01)     *G06K 9/00* (2006.01)
*G01N 33/50* (2006.01)     *G06T 7/00* (2017.01)

(21) Application number: **14183079.4**

(22) Date of filing: **31.05.2012**

(54) **Embryo quality assessment based on blastomere cleavage and morphology**

Embryonenqualitätsbeurteilung auf der Basis von Blastomerspaltung und Morphologie

Évaluation de la qualité des embryons basée sur le clivage des blastomères et morphologie

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.05.2011 US 201161491483 P**

(43) Date of publication of application:
**10.12.2014 Bulletin 2014/50**

(62) Document number(s) of the earlier application(s) in accordance with Art. 76 EPC:
**12728169.9 / 2 714 895**

(73) Proprietor: **Unisense FertiliTech A/S**
**8200 Aarhus N (DK)**

(72) Inventors:
• **Ramsing, Niels B**
  **8240 Risskov (DK)**
• **Hilligsoe, Karen Marie**
  **8000 Aarhus C (DK)**
• **Escrivá, Marcos Meseguer**
  **46015 Valencia (ES)**

(74) Representative: **D Young & Co LLP**
**120 Holborn**
**London EC1N 2DY (GB)**

(56) References cited:
**EP-A1- 2 282 210     WO-A1-2011/025736**

• HERRERO J ET AL: "Linking successful implantation with the exact timing of cell division events obtained by time-lapse system in the embryoscope", FERTILITY AND STERILITY, ELSEVIER SCIENCE INC, NEW YORK, NY, USA, vol. 94, no. 4, 1 September 2010 (2010-09-01), page S149, XP027250457, ISSN: 0015-0282 [retrieved on 2010-08-30]
• LEMMEN J G ET AL: "Kinetic markers of human embryo quality using time-lapse recordings of IVF/ICSI-fertilized oocytes", REPRODUCTIVE BIOMEDICINE ONLINE, REPRODUCTIVE HEALTHCARE LTD, GB, vol. 17, no. 3, 1 January 2008 (2008-01-01), pages 385-391, XP003028429, ISSN: 1472-6483 [retrieved on 2008-07-30]
• M. MESEGUER ET AL: "The use of morphokinetics as a predictor of embryo implantation", HUMAN REPRODUCTION, vol. 26, no. 10, 9 August 2011 (2011-08-09), pages 2658-2671, XP055033123, ISSN: 0268-1161, DOI: 10.1093/humrep/der256

Remarks:
The file contains technical information submitted after the application was filed and not included in this specification

**Description**

The present invention relates to a method and to a system for selecting embryos for in vitro fertilization based on the timing, and duration of observed cell cleavages and associated cell morphology.

**Background**

Infertility affects more than 80 million people worldwide. It is estimated that 10% of all couples experience primary or secondary infertility (Vayena et al. 2001). In vitro fertilization (IVF) is an elective medical treatment that may provide a couple who has been otherwise unable to conceive a chance to establish a pregnancy. It is a process in which eggs (oocytes) are taken from a woman's ovaries and then fertilized with sperm in the laboratory. The embryos created in this process are then placed into the uterus for potential implantation. To avoid multiple pregnancies and multiple births, only a few embryos are transferred (normally less than four and ideally only one (Bhattacharya et al. 2004)). Selecting proper embryos for transfer is a critical step in any IVF-treatment. Current selection procedures are mostly entirely based on morphological evaluation of the embryo at different timepoints during development and particularly an evaluation at the time of transfer using a standard stereomicroscope. However, it is widely recognized that the evaluation procedure needs qualitative as well as quantitative improvements.

One approach is to use 'early cleavage' to the 2-cell stage, (i.e. before 25 - 27 h post insemination/injection), as a quality indicator. In this approach the embryos are visually inspected 25 - 27 hours after fertilization to determine if the first cell cleavage has been completed. However, although the early cleavage as well as other early criteria may be a quality indicator for development into an embryo there is still a need for quality indicators for implantation success and thereby success for having a baby as a result.

WO2011025736 discloses methods, compositions and kits for determining the developmental potential of one or more embryos or pluripotent cells and/or the presence of chromosomal abnormalities in one or more embryos or pluripotent cells are provided. These methods, compositions and kits find use in identifying embryos and oocytes in vitro that are most useful in treating infertility in humans.

Herrero J et al in "Linking successful implantation with the exact timing of cell division events obtained by time-lapse system in the embryoscope", Fertility And Sterility, Elsevier Science Inc, New York, NY, USA, vol. 94, no. 4, page S149 discloses a time-lapse system used for image acquisition of embryos and subsequent analysis of the exact timing of cell division events. The purpose of the study is to link successful implantation of embryos to the exact timing of cell division events.

Lemmen J G et al in "Kinetic markers of human embryo quality using time-lapse recordings of IVF/ICSI-fertilized oocytes", Reproductive Biomedicine Online, Reproductive Healthcare Ltd, GB, vol. 17, no. 3, pages 385 - 391 discloses a time-lapse system used to study the timing and coordination of events during early development from zygote to cleavage stage embryo. The aim was to identify markers linked to good-quality embryos and implantation. Early disappearance of pronuclei and onset of first cleavage after fertilization was correlated with a higher number of blastomeres on day 2 after oocyte retrieval. In addition, synchrony in appearance of nuclei after the first cleavage was significantly associated with pregnancy success (P < 0.05).

EP2282210 discloses a system and method for determining embryo quality comprising monitoring the embryo for a time period, said time period having a length sufficient to comprise at least one cell division period and at least a part of an inter-division period, and determining the length of the at least one cell division period; and/or ii) determining the extent and/or spatial distribution of cellular or organelle movement during the cell division period; and/or iii) determining duration of an inter-division period; and/or iv) determining the extent and/or spatial distribution of cellular or organelle movement during the inter-division period thereby obtaining an embryo quality measure. Thus, the selection of optimal embryos to be implanted after in vitro fertilization (IVF) is facilitated based on the timing, duration, spatial distribution, and extent of observed cell divisions and associated cellular and organelle movement.

M. Meseguer et al, "The Use of Morphokinetics as a Predictor of Embryo Implantation", Human Reproduction, no. 10, ISSN 0268-1161, page 2658 - 2671 discloses an IVF incubator with a built-in camera designed to automatically acquire images at defined time points to simultaneously monitor 72 embryos without removing the embryos from the controlled environment. Analysis of cleavage times, blastomere size and multinucleation was made for 247 transferred embryos with either failed or full implantation. Several parameters were found to be correlated with subsequent implantation (e.g. time of first and subsequent cleavages as well as the time between cleavages).

**Summary of invention**

Aspects and features of the present invention are defined in the appended claims.

Previous studies have often focused on the embryo development before embryonic genome activation. However, the present inventors have found that monitoring the timing and duration of the subsequent cleavages, wherein embryonic genome activation takes place, may lead to additional quality criteria (sometimes referred to as "late phase criteria"), that are very useful in the selection of embryos in order to increase implantation success.

Accordingly, the present invention relates to a method and to a system to facilitate the selection of optimal embryos to be transferred for implantation after in vitro fertilization (IVF) based on the timing, and duration of observed cell cleavages.

## Drawings

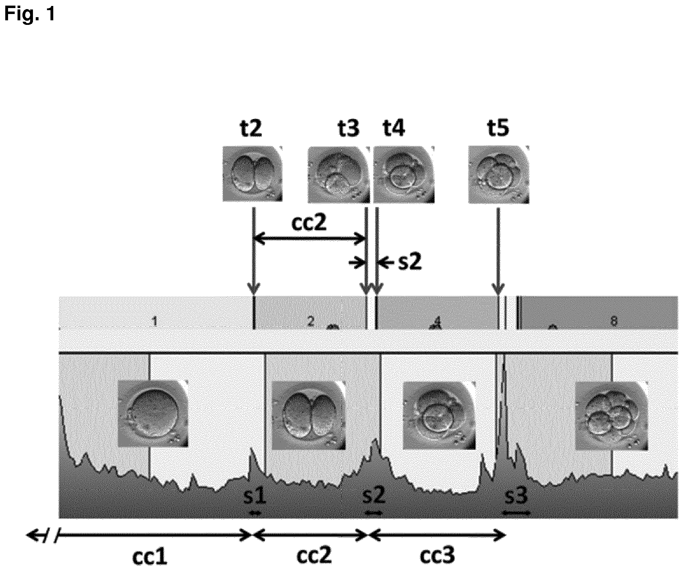

Fig. 1. Nomenclature for the cleavage pattern showing cleavage times (t2-t5), duration of cell cycles (cc1-cc3), and synchronies (s1-s3) in relation to images obtained.

Fig. 2. Hierarchical decision tree with the parameters t5-s2-cc2

Fig. 3 Schematic hierarchical decision tree with the parameters t5-s2-cc2 based on: i) Morphological screening; ii) absence of exclusion criteria; iii) timing of cell division to five cells (t5); iv) synchrony of divisions from 2-cell to 4-cell stage, s2, i.e. duration of 3-cell stage; v) duration of second cell cycle, cc2, i.e. time between division to 3-cell stage and division to 5-cell stage. The classification generates ten grades of embryos with increasing expected implantation potential (right to left) and almost equal number of embryos in each.

Fig. 4. t2:time of cleavage to 2 blastomere embryo

Fig.5. A series of images showing direct cleavage to 3 blastomere embryo. Cleavage from 1 to 3 cells happens in one frame

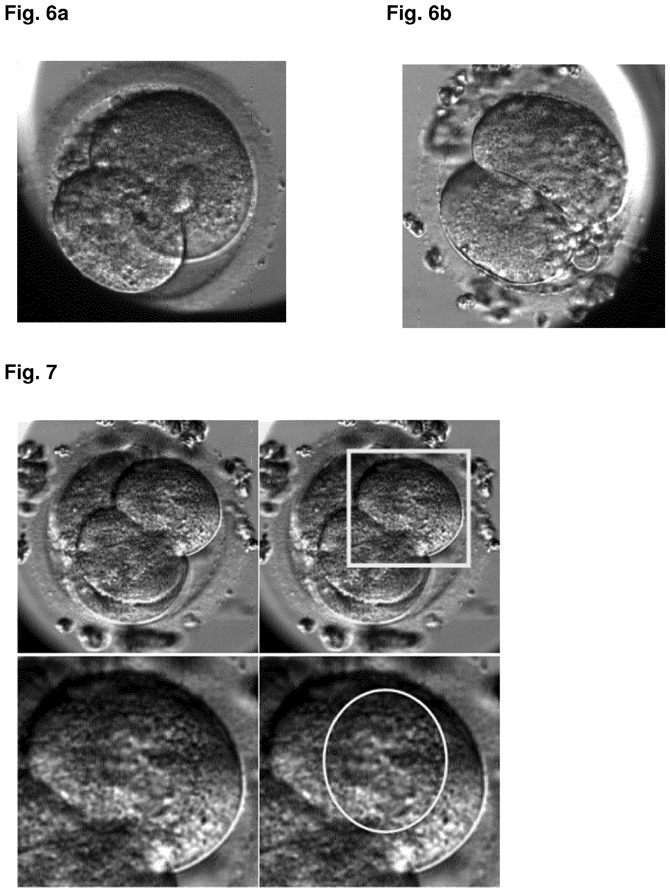

Fig. 6a Uneven blastomere size at the 2 cell stage (2nd cell cycle) - Fig. 6b Even blastomere size at the 2 cell stage

Fig. 7. Multinucleated blastomere at 4 cell stage

Fig. 8. Percentage of embryos having completed a cell division by a given time after fertilization. Blue curves present implanting embryos, red curves represent embryos that do not implant. Four curves of each color represent completion of the four consecutive cell divisions from one to five cells i.e. t2, t3, t4, and t5.

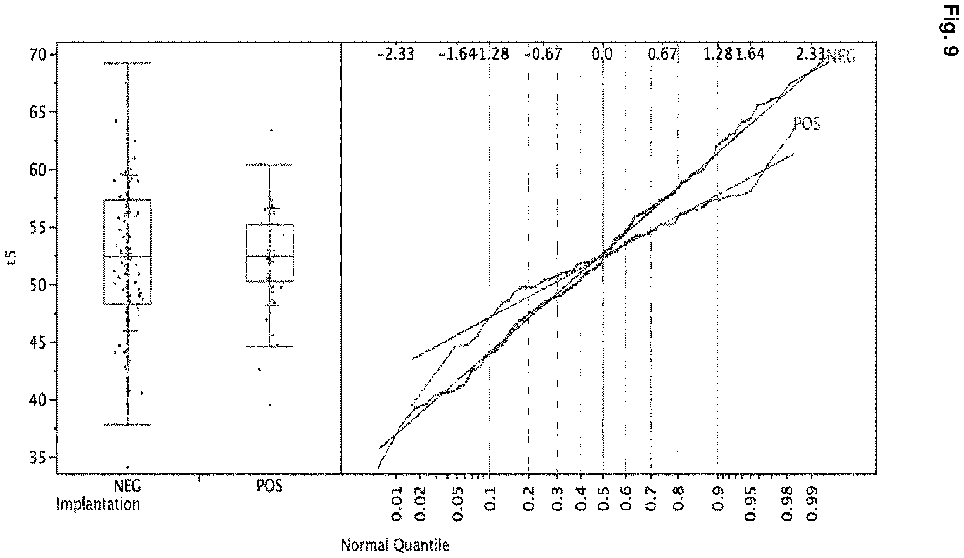

Fig. 9. Distribution of the timing for cell division to five cells, t5, for 61 implanting embryos (positive, blue dots) and for 186 non-implanting embryos (negative, red dots). The left panel show the overall distributions of cleavage times. Short blue lines demarcate standard deviations, means and 95% confidence limits for the mean. Red boxes denote the quartiles for each class of embryos. The right panel show the distribution of observed t5 cleavage times for the two types of embryos (red = non implanted, blue = implanted) plotted as normal quantiles on a plot where a normal distribution is represented by a straight line. The two fitted lines represent normal distributions corresponding to the two types of embryos.

Fig. 10. Percentage of implanting embryos with cell division times inside or outside ranges defined by quartile limits for the total dataset. The three panels show ranges and implantation for: i) division to 2-cells, t2; ii) division to 3-cells, t3; and division to 5-cells, t5. As the limits for the ranges were defined as quartiles, each column represent the same number of transferred embryos with known implantation outcome, but the frequency of implantation was significantly higher for embryos within the rages as opposed to those outside the ranges.

Fig. 11. Percentage of implanting embryos with cell division parameters below or above the median values. The two panels show classification for: i) duration of second cell cycle, cc2; ii) synchrony of divisions from 2-cell to 4-cell stage, s2. As the limits are defined as median values for all 247 investigated embryos with known implantation outcome, each column represent the same number of transferred embryos and the frequency of implantation was significantly higher for embryos with parameter values below the median.

Fig. 12. Implantation rate in high and low implantation groups for the parameters t2, t3, t4, t5, cc2, cc3, and s2.

Fig. 13. Known Implantation data (see example 2) divided into quartiles with respect to t2 and with the expected value for each quartile (left graph). From these quartile groups a new target group is formed by the three neighboring quartiles Q1, Q2 and Q3 having similar probabilities (right graph) - see example 2.

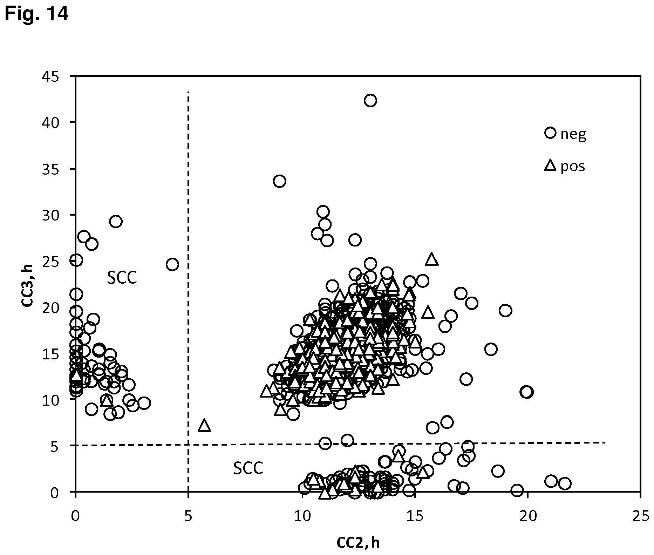

Fig. 14. KID data with successful implantations (triangles) and unsuccessful implantations (circles) for cc2 and cc3 illustrating the usefulness of exclusion criteria.

Fig. 15. Decision tree model built using quality and exclusion criteria from t2 and forward.

Fig. 16. Decision tree model built using quality and exclusion criteria from t4 and forward (i.e. only late phase criteria).

**Definitions**

Cleavage time is defined as the first observed timepoint when the newly formed blastomeres are completely separated by confluent cell membranes, the cleavage time is therefore the time of completion of a blastomere cleavage. In the present context the times are expressed as hours post IntraCytoplasmic Sperm Injection (ICSI) microinjection, i.e. the time of fertilization. Thereby the cleavage times are as follows:

- t2: Time of cleavage to 2 blastomere embryo
- t3: Time of cleavage to 3 blastomere embryo
- t4: Time of cleavage to 4 blastomere embryo
- t5: Time of cleavage to 5 blastomere embryo
- t6: Time of cleavage to 6 blastomere embryo
- t7: Time of cleavage to 7 blastomere embryo
- t8: Time of cleavage to 8 blastomere embryo

Duration of cell cycles is defined as follows:

- cc1 = t2: First cell cycle.
- cc2 = t3-t2: Second cell cycle, duration of period as 2 blastomere embryo.
- cc2b = t4-t2: Second cell cycle for both blastomeres, duration of period as 2 and 3 blastomere embryo.
- cc3 = t5-t3: Third cell cycle, duration of period as 3 and 4 blastomere embryo.
- cc2_3 = t5-t2: Second and third cell cycle, duration of period as 2, 3 and 4 blastomere embryo.
- cc4 = t9-t5: Fourth cell cycle, duration of period as 5, 6, 7 and 8 blastomere embryo.

Synchronicities are defined as follows:

- s2 = t4-t3: Synchrony in division from 2 blastomere embryo to 4 blastomere embryo.
- s3 = t8-t5: Synchrony in division from 4 blastomere embryo to 8 blastomere embryo.
- s3a = t6-t5; s3b = t7-t6; s3c = t8-t7: Duration of the individual cell divisions involved in the development from 4 blastomere embryo to 8 blastomere embryo.

Cleavage period: The period of time from the first observation of indentations in the cell membrane (indicating onset of cytoplasmic cleavage) to the cytoplasmic cell cleavage is complete so that the blastomeres are completely separated by confluent cell membranes. Also termed as duration of cytokinesis.

Fertilization and cleavage are the primary morphological events of an embryo, at least until the 8 blastomere stage. Cleavage time, cell cycle, synchrony of division and cleavage period are examples of morphological embryo parameters that can be defined from these primary morphological events and each of these morphological embryo parameters are defined as the duration of a time period between two morphological events, e.g. measured in hours.

A normalized morphological embryo parameter is defined as the ratio of two morphological embryo parameters, e.g. cc2 divided by cc3 (cc2/cc3), or cc2/cc2_3 or cc3/t5 or s2/cc2.

The following discrete (binary) variables can be used

- MN2: Multi nucleation observed at the 2 blastomere stage; can take the values "True" or False".
- MN4: Multi nucleation observed at the 4 blastomere stage; can take the values "True" or False".
- EV2: Evenness of the blastomeres in the 2 blastomere embryo; can take the values "True" (i.e. even) or "False" (i.e. uneven).

Rearrangement of cellular position = Cellular movement (see below)

Cellular movement: Movement of the center of the cell and the outer cell membrane. Internal movement of organelles within the cell is NOT cellular movement. The outer cell membrane is a dynamic structure, so the cell boundary will continually change position slightly. However, these slight fluctuations are not considered cellular movement. Cellular movement is when the center of gravity for the cell and its position with respect to other cells change as well as when cells divide. Cellular movement can be quantified by calculating the difference between two consecutive digital images of the moving cell. An example of such quantification is described in detail in the pending PCT application entitled "Determination of a change in a cell population", filed Oct 16 2006. However, other methods to determine movement of the cellular center of gravity, and/or position of the cytoplasm membrane may be envisioned e.g. by using FertiMorph software (ImageHouse Medical, Copenhagen, Denmark) to semi-automatically outline the boundary of each blastomere in consecutive optical transects through an embryo.

Organelle movement: Movement of internal organelles and organelle membranes within the embryo which may be visible by microscopy. Organelle movement is not Cellular movement in the context of this application.

Movement: spatial rearrangement of objects. Movements are characterized and/or quantified and/or described by many different parameters including but restricted to: extent of movement, area and/or volume involved in movement, rotation, translation vectors, orientation of movement, speed of movement, resizing, inflation/deflation etc. Different measurements of cellular or organelle movement may thus be used for different purposes some of these reflect the extent or magnitude of movement, some the spatial distribution of moving objects, some the trajectories or volumes being afflicted by the movement.

Embryo quality is a measure of the ability of said embryo to successfully implant and develop in the uterus after transfer. Embryos of high quality will successfully implant and develop in the uterus after transfer whereas low quality embryos will not.

Embryo viability is a measure of the ability of said embryo to successfully implant and develop in the uterus after transfer. Embryos of high viability will successfully implant and develop in the uterus after transfer whereas low viability embryos will not. Viability and quality are used interchangeably in this document

Embryo quality (or viability) measurement is a parameter intended to reflect the quality (or viability) of an embryo such that embryos with high values of the quality parameter have a high probability of being of high quality (or viability), and low probability of being low quality (or viability). Whereas embryos with an associated low value for the quality (or viability) parameter only have a low probability of having a high quality (or viability) and a high probability of being low quality (or viability)

**Detailed description of invention**

**Determination of quality**

The search for prognostic factors that predict embryo development and the outcome of in vitro fertilization (IVF) treatment have attracted considerable research attention as it is anticipated that knowledge of such factors may improve future IVF treatments.

As discussed above one promising predictive factor is the precise timing of key events in early embryo development. Studies that involve imaging have been limited to measurements of early development, such as pronuclear formation and fusion, and time to first cleavage (Nagy, Z.P. 1994, Fenwick, J. 2002, Lundin, K. 2001, Lemmen, J.G. 2008). An important finding of the time-lapse analysis is a correlation between the early cleavage pattern to the 4-cell stage and subsequent development to the blastocyst stage. Morphokinetic analysis on the development of bovine embryos have also been published, where timing, duration and intervals between cell cleavages in early embryo development successfully predicted subsequent development to the expanded blastocyst stage (Ramsing 2006, Ramsing 2007).

The present inventors have performed a large clinical study involving many human embryos and monitoring the development, not only until formation of a blastocyst, but further until sign of implantation of the embryo. In this study important differences in the temporal patterns of development between the embryos that implanted (i.e. embryos that were transferred and subsequently led to successful implantation) and those that did not (i.e. embryos that were transferred but did not lead to successful implantation) were observed.

It has been found that there exists an optimal time range for parameters characterizing the embryonic cell divisions. Embryos which cleave at intermediate timepoints have significantly improved chance of ongoing implantation when compared with embryos that either developed faster or slower. The observations support the hypothesis that the viability of embryos is associated with a highly regulated sequence of cellular events that begin at the time of fertilization. In this large clinical study on exclusively good quality embryos, it has been confirmed that an embryo's capability to implant is correlated with numerous different cellular events, e.g. timing of cell divisions and time between divisions, as well as uneven blastomere size and multinucleation. The complexity, structure and parameters in the models must be adaptable to different clinical situations like incubation temperature, transfer times, culture media and other.

Timing of early events in embryonic development correlates with development into a blastocyst, however it has been found that the development into a blastocyst does not necessarily correlate with successful implantation of the embryo. By using implantation as the endpoint, not only embryo competence for blastocyst formation, but also subsequent highly essential processes such as hatching and successful implantation in the uterus is assessed.

Thus, the data allows the detection of later developmental criteria for implantation potential. The results in particular indicate that timing of later events such as the cleavage to the five cell stage are a consistently good indicator of implantation potential, and that the discrimination between implanting and non-implanting embryos is improved when using the later cell division events, e.g. t5 as opposed to the earlier events (t2, t3 and t4). The presented data indicate that incubating the embryos to day 3, which enables evaluation of timing for cell divisions from five to eight cells, after completion of the third cell cycle, can give additional important information that will improve the ability to select a viable embryo with high implantation potential.

Accordingly, in a first aspect the invention relates to a method for determining embryo quality comprising monitoring the embryo for a time period, and determining one or more quality criteria for said embryo, and based on said quality criteria determining the embryo quality. In the present context, the embryo quality is a quality relating to implantation success.

The selection criteria (quality criteria) can be based on single variables, composite variables (variables that can be calculated from other variables) and multiple variables (more variables at once).

The quality criteria used herein are preferably criteria relating to the phase from a 2 to 8 blastomere embryo, in particularly from 4 to 8 blastomere embryo, and accordingly, the present quality criteria may be determination of the time for cleavage into a 5 blastomere embryo, 6 blastomere embryo, 7 blastomere embryo, and/or 8 blastomere embryo.

The present quality criteria is a preferably criteria obtained within the time period of from 48 to 72 hours from fertilisation. As discussed above, the clock starts at the time of fertilisation which in the present context is meant to be the time of injection of the sperm, such as by ICSI microinjection. Preferably the embryo is monitored for a time period comprising at least three cell cycles, such as at least four cell cycles.

In particular it has been found that the time for cleavage into a 5 blastomere embryo has an important impact on the implantation success, and therefore the quality criteria is preferably determination of the time for cleavage into a 5 blastomere embryo, i.e. t5. As shown below t5 should preferably be in the range of from 47-58 hours from fertilisation, more preferably in the range of 48-57 hours from fertilisation, more preferably in the range of 48.7 - 55.6 hours from fertilisation.

The time for cleavage into a 2 blastomere embryo has an important impact on the implantation success and t2 should preferably be less than 32 hours from fertilisation, more preferably less than 27.9 hours from fertilisation. In a further embodiment of the invention $t2 \geq 24.3$ hours.

The time for cleavage into a 3 blastomere embryo may have an impact on the implantation success and t3 should preferably be less than or equal to 40.3 hours from fertilisation. In a further embodiment of the invention $t3 \geq 35.4$ hours.

The time for cleavage into a 6 blastomere embryo may have an impact on the implantation success and t6 should preferably be less than 60 hours from fertilisation.

The time for cleavage into a 7 blastomere embryo may have an impact on the implantation success and t7 should preferably be less than 60 hours from fertilisation.

The time for cleavage into an 8 blastomere embryo may have an impact on the implantation success and t8 should preferably be less than 60 hours from fertilisation more preferably less than 57.2 hours from fertilisation.

The duration of the period as a 2 blastomere embryo, i.e. the second cell cycle cc2 = t3-t2, may have an impact on the implantation success and cc2 should preferably be less than 12.7 hours.

The duration of the period as a 2 and 3 blastomere embryo, i.e. the second cell cycle for both blastomeres cc2b = t4-t2, may have an impact on the implantation success and cc2 should preferably be less than 12.7 hours. In a further embodiment of the invention cc2 > 5 hours.

The duration of the period as a 3 and 4 blastomere embryo, i.e. cc3 = t5-t3, may have an impact on the implantation success and cc3 should preferably be less than or equal to 16.3 hours. In a further embodiment of the invention $cc3 \geq 5$ hours or $cc3 \geq 12.9$ hours.

The duration of the period as a 2, 3 and 4 blastomere embryo, i.e. cc2_3 = t5-t2, may have an impact on the implantation success and cc2_3 should preferably be less than or equal to 28.7 hours. In a further embodiment of the invention $cc2\_3 \geq 24$ hours.

The synchrony in division from a 2 blastomere embryo to a 4 blastomere embryo, i.e. s2 = t4-t3, may have an impact on the implantation success and s2 should preferably be less than 1.33 hours or less than 0.33 hours.

The synchrony in division from a 4 blastomere embryo to an 8 blastomere embryo, i.e. s3 = t8-t5, may have an impact on the implantation success and s3 should preferably be less than 2.7 hours.

*Multiple variables*

Multiple variables may be used when choosing selection criteria. When using multiple variables it can be an advantage that the variables are selected progressively such that initially one or more of the variables that can be determined early with a high accuracy are chosen, e.g. t2, t3, t4 or t5. Later other variables that can be more difficult to determine and is associated with a higher uncertainty can be used (e.g. multinuclearity, evenness of cells and later timings (e.g. after t5)).

In addition to t5 other criteria may be added to determine the embryo quality. In one embodiment the present quality criteria is combined with determination of second cell cycle length in order to establish the embryo quality. In another embodiment the present quality criteria is combined with determination of synchrony in cleavage from 2 blastomere embryo to 4 blastomere embryo.

Accordingly, in one embodiment the embryo quality is determined from a combination of determination of time for cleavage to a 5 blastomere embryo and determination of the second cell cycle length.

Furthermore, three different criteria may be combined, for example so that determination of time for cleavage to a 5 blastomere embryo and determination of the second cell cycle length are combined with determination of synchrony in cleavage from 2 blastomere.

*Normalized parameters*

In one embodiment of the invention an embryo quality criterion is selected from the group of normalized morphological embryo parameters, in particular the group of normalized morphological parameters based on two, three, four, five or more parameters selected from the group of t2, t3, t4, t5, t6, t7 and t8. By normalizing the parameters the time of fertilization may be "removed" from the embryo quality assessment. Further, a normalized morphological embryo parameter may better describe the uniformity and/or regularity of the developmental rate of a specific embryo independent of the environmental conditions, because instead of comparing to "globally" determined absolute time intervals that may depend on the local environmental conditions, the use of normalized parameters ensure that specific ratios of time intervals can be compared to "globally" determined normalized parameters, thereby providing additional information of the embryo development. E.g. the ratio cc2/cc3 may indicate whether the duration of cell cycle 2 corresponds (relatively) to the duration of cell cycle 3, cc2/cc2_3 provides the duration of the period as a 2 blastomere embryo relative to the duration of the period as a 2, 3 and 4 blastomere embryo, s2/cc2 provides the synchronicity from 2 to 4 blastomere relative to the duration of the period as a 2 blastomere embryo and cc3/t5 provides the duration of cell cycle 3 relative to the time of cleavage to a 5 blastomere embryo.

In one embodiment of the invention the normalized morphological embryo parameter cc2/cc2_3 = 1 - cc3/cc2_3 = (t3-t2)/(t5-t2) should be between 0.41 and 0.47.

In one embodiment of the invention the normalized morphological embryo parameter cc3/t5 = 1 - t3/t5 should be greater than 0.3 or between 0.26 and 0.28.

In one embodiment of the invention the normalized morphological embryo parameter s2/cc2 = (t4-t3)/(t3-t2) should be less than 0.025.

In one embodiment of the invention the normalized morphological embryo parameter s3/cc3 = (t8-t5)/(t5-t3) should be less than 0.18.

In one embodiment of the invention the normalized morphological embryo parameter cc2/cc3 = (t3-t2)/(t5-t3) should be between 0.72 and 0.88.

*Irregularity from 4 to 8 blastomeres*

The timing of the individual cell divisions when the embryo develops from 4 to 8 blastomeres (i.e. s3a=t6-t5, s3b=t7-t6 and s3c=t8-t7) may be associated with embryo quality and success of implantation. These timings may demonstrate the competence of each individual cell to perform a cell division. Possible irregularities or abnormalities in the mitosis may result in large differences between the value of s3a, s3b and/or s3c. Thus, in a further embodiment of the invention an embryo quality criterion is the irregularity of the timing of cell divisions, such as irregularity of the timing of cell divisions until the 8 blastomere embryo, such as irregularity of the timing of cell divisions when developing from 4 to 8 blastomere embryo. In a further embodiment of the invention an embryo quality criterion is the maximum of s3a, s3b and s3c. Preferably the maximum of s3a, s3b and s3c is less than 1.5 hours. In a further embodiment of the invention an embryo quality criterion is the maximum of s3a, s3b and s3c divided by s3, preferably max(s3a, s3b, s3c)/s3 is less than 0.5. Please note that max(s3a, s3b, s3c)/s3 is a normalized morphological embryo parameter based on t5, t6, t7 and t8.

Multi nucleation may be an embryo quality parameter, in particular multi nucleation observed at the 4 blastomere stage (MN4). Preferably no multi nucleation should be present at the 4 blastomere stage, thus preferably MN4 = False.

Even size of the blastomeres may be an embryo quality parameter, in particular a two blastomere embryo should have blastomeres of even size, thus preferably EV2 = True.

EV2: Evenness of the blastomeres in the 2 blastomere embryo; can take the values "True" (i.e. even) or "False" (i.e. uneven).

*Exclusion criteria*

An embryo population may be subject to one or more exclusion criteria in order to exclude embryos from the population with a low probability of implantation success, i.e. the outliers. This may be embryos that fulfil many of the positive selection criteria but show unusual behaviour in just one or two selection criteria. Examples of exclusion criteria are the discrete criteria such as blastomere evenness at t2 and multi nuclearity at the four-blastomere stage. However, exclusion criteria may also be applied to the morphological embryo parameters. It has long been known that slowly developing embryos are an indication of poor quality, reflected in a very high value of t2 (>31.8 hours), but cleavage from one blastomere directly to three blastomeres may also be an indication of a poor quality embryo associated with low implantation rate. This may be reflected in very low values for cc2 and cc3.

A specific exclusion criterion pointing out a group of embryos in a population with a low probability of implantation does not imply that the rest of the population has a high probability of implantation. An exclusion criterion only indicates poor quality embryos. Thus, in one embodiment of the invention said one or more quality criteria are combined with one or more exclusion criteria. An example of applying exclusion criteria to a population of embryos (based on KID data, see example 2) is shown in fig. 17. cc2 (hours) is along the x-axis whereas cc3 (hours) is along the y-axis. Embryos that successfully implanted are depicted as triangles whereas non-successful embryos (embryos that did not successfully implant) are depicted as circles. It is seen that a large group of non-successful embryos with low cc2 assemble to the left in the figure and a large group of non-successful embryos with low cc3 assemble in the bottom of the figure. If exclusion criteria of cc2 less than 5 hours and/or cc3 less than 5 hours are applied, large groups of non-successful embryos can be excluded thereby helping to isolate the successful embryos, from where better quality criteria can be extracted.

*Monitoring*

The embryo is monitored regularly to obtain the relevant information, preferably at least once per hour, such as at least twice per hour, such as at least three times per hour. The monitoring is preferably conducted while the embryo is situated in the incubator used for culturing the embryo. This is preferably carried out through image acquisition of the embryo, such as discussed below in relation to time-lapse methods.

Determination of selection criteria's can be done for example by visual inspection of the images of the embryo and/or by automated methods such as described in detail in the pending PCT application entitled "Determination of a change in a cell population" filed Oct 16 2006. Furthermore, other methods to determine selection criteria's can be done by determining the position of the cytoplasm membrane by envisioned e.g. by using FertiMorph software (ImageHouse Medicall Copenhagen, Denmark). The described methods can be used alone or in combination with visual inspection of the images of the embryo and/or with automated methods as described above.

*Decision tree model*

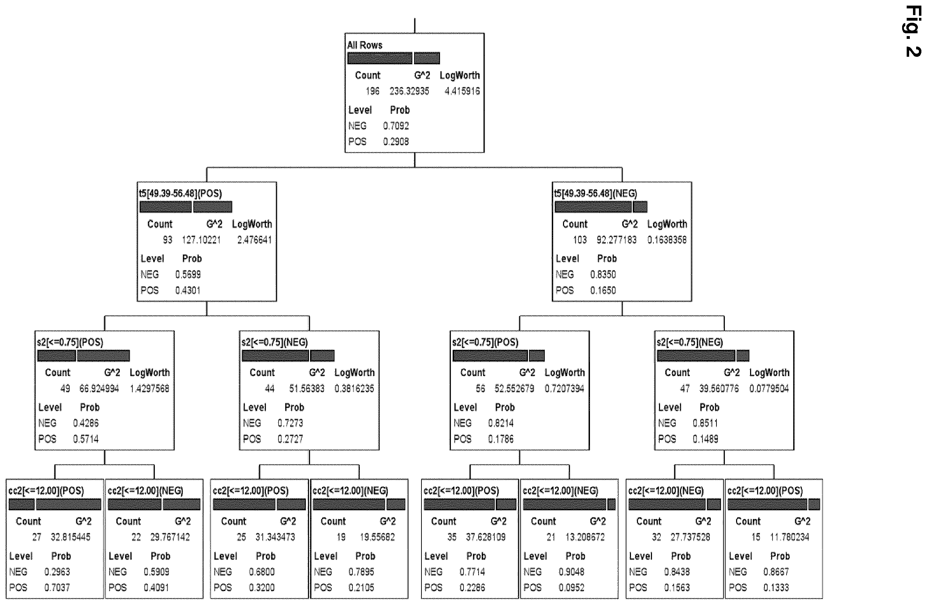

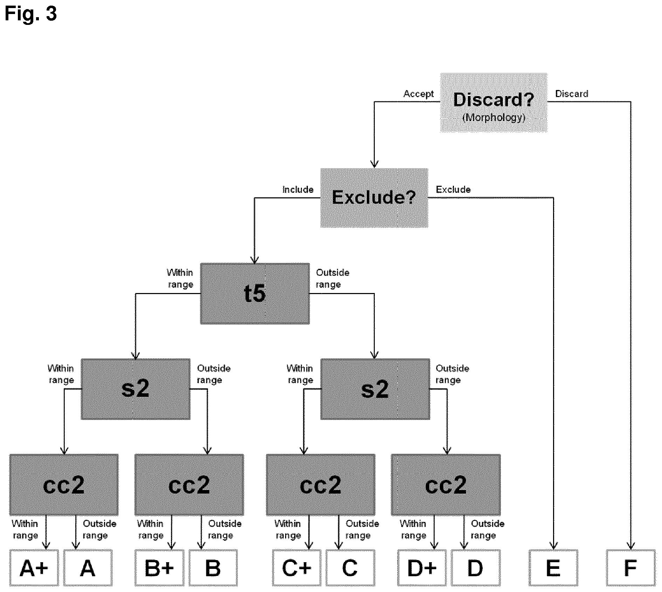

In particularly, the criteria may be combined in a hierarchical form, as shown in Figs. 2 and 3, see also example 1 for more information thereby giving rise to a decision tree model (or classification tree model) to select embryos with higher implantation probabilities. In a classification tree model several variables are used to split the embryos into groups with different associated probability of implantation success rate by using successive splitting rules. The classification tree model can be optimized under a set of given constraints selecting the optimal variables to use in the splitting rules from a set of possible variables. The variables used in the model can e.g. be morphological embryo parameters based on time intervals between morphological events and the corresponding normalized morphological embryo parameters and discrete variables (e.g. multi nuclearity or evenness of blastomeres), or any combination of these variables. This type of models can be evaluated using area under the ROC curve (AUC). AUC is 0.5 if no splitting is applied and the splitting improves the predictive power if AUC > 0.5.

The decision tree depicted in Fig. 3 represents a sequential application of the identified selection criteria in combination with traditional morphological evaluation.

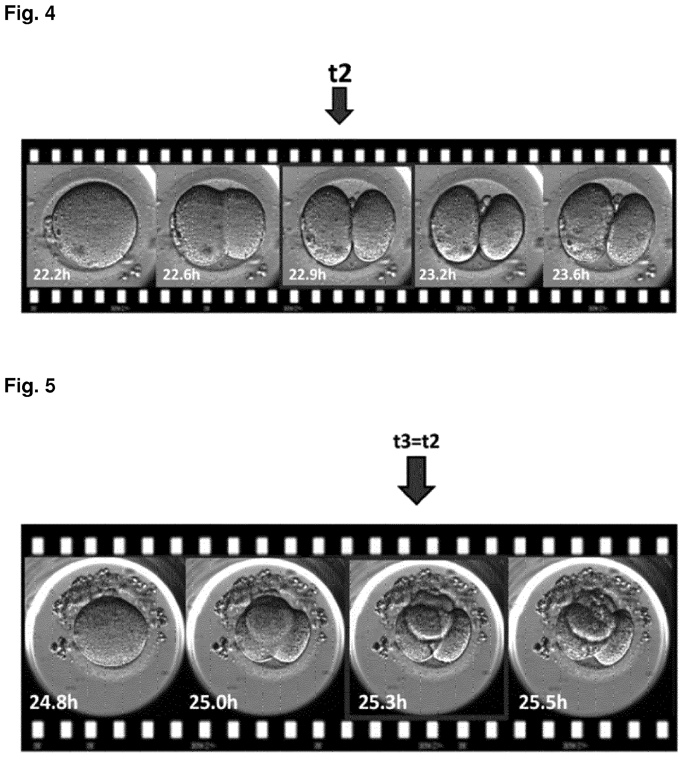

The decision tree subdivided embryos into 6 categories from A to F. Four of these categories (A to D) were further subdivided into two sub-categories (+) or (-) as shown in Figure 5, giving a total of 10 categories. The hierarchical decision procedure start with a morphological screening of all embryos in a cohort to eliminate those embryos that are clearly NOT viable (i.e. highly abnormal, attretic or clearly arrested embryos). Those embryos that are clearly not viable are discarded and not considered for transfer (category F). Next step in the model is to exclude embryos that fulfil any of the three exclusion criteria: i) uneven blastomere size at the 2 cell stage, ii) abrupt division from one to three or more cells; or iii) multi-nucleation at the four cell stage (category E). The subsequent levels in the model follow a strict hierarchy based on the binary timing variables t5, s2 and cc2. First, if the value of t5 falls inside the optimal range the embryo is categorized as A or B. If the value of t5 falls outside the optimal range (or if t5 has not yet been observed at 64 hours) the embryo is categorized as C or D.

If the value of s2 falls inside the optimal range ($\leq 0.76$ hrs) the embryo is categorized as A or C depending on t5 and similarly if the value of s2 falls outside the optimal range the embryo is categorized as B or D depending on t5.

Finally, the embryo is categorized with the extra plus (+) if the value for cc2 is inside the optimal range ($\leq 11.9$ hrs) (A+/B+/C+/D+) and is categorized as A,B,C,D if the value for cc2 is outside the optimal range.

In the study discussed in example 1, the decision procedure divides all the 247 evaluated embryos in ten different categories containing approx. the same number of transferred embryos but with largely decreasing implantation potential (i.e. from 68% for A+ to 8% for E).

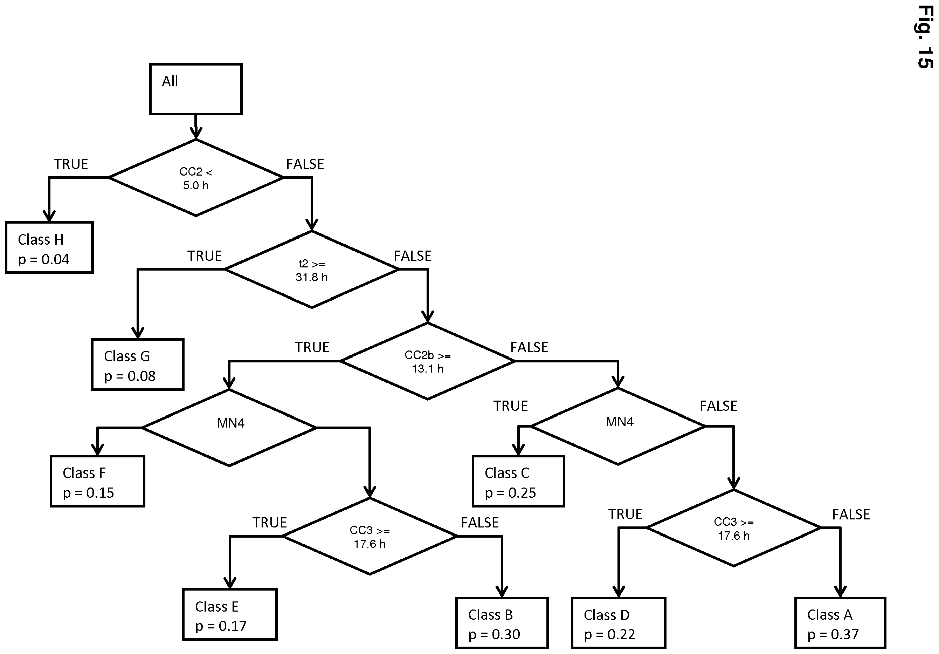

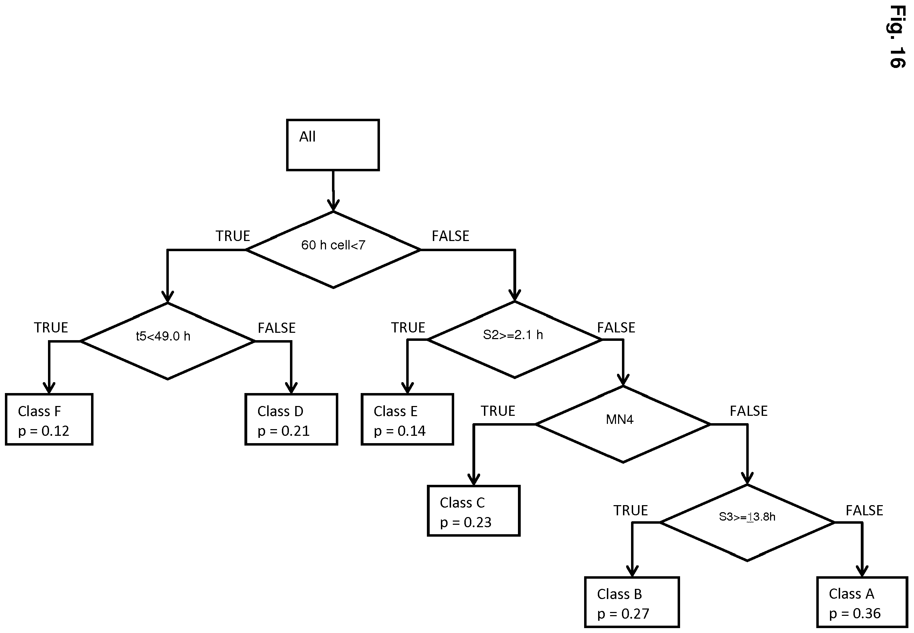

Decision tree models have also been constructed based on KID data from 1598 human embryos (see example 2). The two decision trees are based on quality and exclusion criteria from t2 onwards (fig. 15) and from t4 onwards (fig. 16). By means of these decision tree models the 1598 embryos have been classified into eight quality classes A-H ranging from an implantation probability of 0.04 to 0.37 (fig. 15) and into six classes A-F ranging from an implantation probability of 0.12 to 0.36 (fig. 16). This should be compared to a total implantation probability for all 1598 embryos of 0.28. These probabilities only apply to this specific data set and cannot be applied to IVF embryos in general. The probability of implantation of a specific embryo from a specific woman depends on many other parameters. However, this dataset provides a unique opportunity to test the quality and exclusion criteria presented herein in order to optimize the classification of IVF embryos. E.g. to classify (in terms of quality) a number of embryos taken from a single woman in order to select the best embryo(s) for transfer. Possibly none of the embryos from a single woman fulfils all optimal quality criteria because all embryos are mediocre or poor quality. However, a transfer must be performed and a classification of the embryos is therefore important to select the best of embryos.

*Combination with measurements of movement*

The quality criteria discussed above may also be combined with determinations of movement of the embryo, such as i) determining the extent and/or spatial distribution of cellular or organelle movement during the cell cleavage period; and/or ii) determining the extent and/or spatial distribution of cellular or organelle movement during the inter-cleavage period thereby obtaining an embryo quality measure.

Volumes within the zona pelucida that are devoid of movement (or similarly areas in a projected 2D image of the embryo that remain stationary) are an indication of "dead" zones within the embryo. The more and larger these immotile "dead" zones the lower the probability of successful embryo development. Large areas within a time-lapse series of embryo images without any type of movement (i.e. neither cellular nor organelle movement) indicates low viability. Organelle movement should generally be detectable in the entire embryo even when only comparing two or a few consecutive frames. Cellular movement may be more localized especially in the later phases of embryo development.

The cell positions are usually relatively stationary between cell cleavages (i.e. little cellular movement), except for a short time interval around each cell cleavage, where the cleavage of one cell into two leads to brief but considerable rearrangement of the dividing cells as well as the surrounding cells (i.e. pronounced cellular movement). The lesser movement between cleavages is preferred.

In one embodiment, in order to determine movement relating to either cleavage and inter-cleavage periods, the length of each cleavage period may be determined as well as the length of each inter-cleavage period. Preferably the period of cellular movement in at least two inter-cleavage periods is determined as well as the extent of cellular movement in at least two inter-cleavage periods. Furthermore, it has been found that rapid cleavage seems to increase quality of the embryo, where rapid normally means less than 2 hours.

In relation to movement during cleavage and inter-cleavage periods we also refer to PCT application WO 2007/144001.

A neural network or other quantitative pattern recognition algorithms may be used to evaluate the complex cell motility patterns described above, for example using different mathematical models (linear, Princepal component analysis, Markov models etc.)

*Time-lapse monitoring*

A particular use of the invention is to evaluate image series of developing embryos (time-lapse images). These time-lapse images may be analyzed by difference imaging equipment (see for example WO 2007/042044 entitled "Determination of a change in a cell population"). The resulting difference images can be used to quantify the amount of change occurring between consecutive frames in an image series.

The invention may be applied to analysis of difference image data, where the changing positions of the cell boundaries (i.e. cell membranes) as a consequence of cellular movement causes a range parameters derived from the difference image to rise temporarily (see WO 2007/042044). These parameters include (but are not restricted to) a rise in the mean absolute intensity or variance. Cell cleavages and their duration and related cellular re-arrangement can thus be detected by temporary change, an increase or a decrease, in standard deviation for all pixels in the difference image or any other of the derived parameters for "blastomere activity" listed in WO 2007/042044. However the selection criteria may also be applied to visual observations and analysis of time-lapse images and other temporally resolved data (e.g. excretion or uptake of metabolites, changes in physical or chemical appearance, diffraction, scatter, absorption etc.) related to embryo.

Of particular interest are the onset, magnitude and duration of cell cleavages that may be quantified as peaks or valleys, in derived parameter values. These extremes, peaks or valleys, frequently denote cell cleavage events. The shape of each peak also provides additional information as may the size of the peak in general. A peak may also denote an abrupt collapse of a blastomer and concurrent cell death. However, it may be possible to separate cell cleavage events and cell death events by the peak shape and change in base values before and after the event. The baseline of most parameters are usually not affected by cell cleavage whereas cell lysis is frequently accompanied by a marked change in the baseline value (for most parameters in a decrease following lysis.)

In summary, the present invention demonstrates that routine time-lapse monitoring of embryo development in a clinical setting (i.e. automatic image acquisition in an undisturbed controlled incubation environment) provide novel information about developmental parameters that differ between implanting and non-implanting embryos.

**Embryo**

In some cases the term "embryo" is used to describe a fertilized oocyte after implantation in the uterus until 8 weeks after fertilization at which stage it becomes a foetus. According to this definition the fertilized oocyte is often called a pre-embryo until implantation occurs. However, throughout this patent application we will use a broader definition of the term embryo, which includes the pre-embryo phase. It thus encompasses all developmental stages from the fertilization of the oocyte through morula, blastocyst stages hatching and implantation.

An embryo is approximately spherical and is composed of one or more cells (blastomeres) surrounded by a gelatine-like shell, the acellular matrix known as the zona pellucida. The zona pellucida performs a variety of functions until the embryo hatches, and is a good landmark for embryo evaluation. The zona pellucida is spherical and translucent, and should be clearly distinguishable from cellular debris.

An embryo is formed when an oocyte is fertilized by fusion or injection of a sperm cell (spermatozoa). The term is traditionally used also after hatching (i.e. rupture of zona pelucida) and the ensuing implantation. For humans the fertilized oocyte is traditionally called an embryo for the first 8 weeks. After that (i.e. after eight weeks and when all major organs have been formed) it is called a foetus. However the distinction between embryo and foetus is not generally well defined.

During embryonic development, blastomere numbers increase geometrically (1-2-4-8-16- etc.). Synchronous cell cleavage is generally maintained to the 16-cell stage in embryos. After that, cell cleavage becomes asynchronous and finally individual cells possess their own cell cycle. Human embryos produced during infertility treatment are usually transferred to the recipient before 16-blastomere stage. In some cases human embryos are also cultivated to the blastocyst stage before transfer. This is preferably done when many good quality embryos are available or prolonged incubation is necessary to await the result of a pre-implantation genetic diagnosis (PGD).

Accordingly, the term embryo is used in the following to denote each of the stages fertilized oocyte, zygote, 2-cell, 4-cell, 8-cell, 16-cell, morula, blastocyst, expanded blastocyst and hatched blastocyst, as well as all stages in between (e.g. 3 -cell or 5-cell)

**Other measurements**

A final analysis step could include a comparison of the made observations with similar observations of embryos of different quality and development competence, as well as comparing parameter values for a given embryo with other quantitative measurements made on the same embryo. This may include a comparison with online measurements such as blastomere motility, respiration rate, amino acid uptake etc. A combined dataset of blastomere motility analysis, respiration rates and other quantitative parameters are likely to improve embryo selection and reliably enable embryologist to choose the best embryos for transfer.

Thus, in one embodiment the method according to the invention may be combined with other measurements in order to evaluate the embryo in question, and may be used for selection of competent embryos for transfer to the recipient.

Such other measurements may be selected from the group of respiration rate, amino acid uptake, motility analysis, blastomere motility, morphology, blastomere size, blastomere granulation, fragmentation, blastomere colour, polar body orientation, nucleation, spindle formation and integrity, and numerous other qualitative measurements. The respiration measurement may be conducted as described in PCT publication no. WO 2004/056265.

**Culture medium**

In a preferred embodiment the observations are conducted during cultivation of the cell population, such as wherein the cell population is positioned in a culture medium. Means for culturing cell population are known in the art. An example of culturing an embryo is described in PCT publication no. WO 2004/056265.

**Data carrier**

The invention further relates to a data carrier comprising a computer program directly loadable in the memory of a digital processing device and comprising computer code portions constituting means for executing the method of the invention as described above.

The data carrier may be a magnetic or optical disk or in the shape of an electronic card as for example the type EEPROM or Flash, and designed to be loaded into existing digital processing means.

**Selection or identification of embryos**

The present invention further provides a method for selecting an embryo for transplantation. The method implies that the embryo has been monitored as discussed above to determine when cell cleavages have occurred.

The selection or identifying method may be combined with other measurements as described above in order to evaluate the quality of the embryo. The important criteria in a morphological evaluation of embryos are: (1) shape of the embryo including number of blastomers and degree of fragmentation; (2) presence and quality of a zona pellu-cida; (3) size; (4) colour and texture; (5) knowledge of the age of the embryo in relation to its developmental stage, and (6) blastomere membrane integrity.

The transplantation may then be conducted by any suitable method known to the skilled person.

**Examples**

**Example 1 - Retrospective study**

*Materials and methods*

The research project was conducted at the Instituto Valenciano de Infertilidad-IVI, Valencia. The procedure and protocol was approved by an Institutional Review Board, (IRB), which regulates and approves database analysis and clinical IVF procedures for research at IVI. The project complies with the Spanish Law governing Assisted Reproductive Technologies (14/2006). The present study included a total of 2903 oocytes from which 2120 embryos were generated in 285 IVF treatment cycles between September 2009 and September 2010. All embryos were obtained after fertilization by Intra Cytoplasmic Sperm Injection (ICSI) and were part of the clinic's standard (n=188) and ovum donation program (n=97). Time-lapse images were acquired of all embryos, but only transferred embryos with known implantation (i.e. either 0% implantation or 100% implantation) were investigated by detailed time-lapse analysis measuring the exact timing of the developmental events in hours-post-fertilization by ICSI.

The exclusion criteria for standard patients and recipients with respect to this study were: low response (less than 5 MII oocytes), endometriosis, Polycystic Ovarian Syndrome (PCOS), hydrosalpynx, BMI > 30 kg/m$^2$, uterine pathology (myomas, adenomyiosis, endocrinopaties, trombophylia, chronic pathologies, acquired or congenital uterine abnormalities), recurrent pregnancy loss, maternal age over 39 years old for standard patients and 45 for oocyte donation recipients (aging uterus), or severe masculine factor (presenting less than 5 million motile sperm cells in total in the ejaculate).

*Ovarian stimulation in standard patients and oocyte donors*

All donors were from the clinic's egg donation program. Only patients having fulfilled the inclusion criteria were included in the study. Briefly, donors were between 18 and 35 years old without current or past exposure to radiation or hazardous chemical substances, drug use, no family history of hereditary or chromosomal diseases, a normal karyotype, and tested negative for fragile X Syndrome and sexually transmitted diseases as stated by Spanish law (Garrido, N. 2002). The mean age of the male patients of the study population was 37.9 years (SD=5.2). The mean age of the female population was 36.9 years (SD=4.9). All donors had normal menstrual cycles of 26-34 days duration, normal weight (BMI of 18-28 kg/m$^2$), no endocrine treatment (including gonadotrophins and oral contraception) in the 3 months preceding the study, normal uterus and ovaries at transvaginal ultrasound (no signs of polycystic ovary syndrome), and antral follicle count (AFC) >20 on the first day of gonadotrophin administration, after downregulation with GnRH agonist (Meseguer, M. 2010).

Prior to controlled ovarian stimulation (COS), cycles with GnRH agonist protocols were used. In GnRH agonist protocols, patients started with administration of 0.5 mg leuprolide acetate (Procrin ®; Abbott, Madrid, Spain) in the midluteal phase of the previous cycle, until negative vaginal ultrasound defined ovarian quiescence. Patients with adequate pituitary desensitization started their stimulation, and the dose of GnRH-agonist was reduced to 0.25 mg per day until the day of hCG administration (Melo, M. 2009).

For COS the treatments proceeded as previously described (Melo, M. 2010). Briefly, donors and patients treated with 150 IU of rFSH (Gonal-f; Merck Serono) plus 75 IU HP-hMG (Menopur; Ferring). The fixed starting dose of 225 IU gonadotropins per day was initiated on day 3 of menstruation and sustained for the first 5 days of controlled ovarian stimulation, during which serum E2 was assessed. The gonadotropin dose was adjusted if necessary. Serial transvaginal ultrasound examinations were initiated on day 5 of controlled ovarian stimulation and were performed every 48 hours to monitor the follicular growth. Human chorionic gonadotropin (hCG) (Ovitrelle, Serono Laboratories, Madrid, Spain) was administered subcutaneously when at least eight leading follicles reached a mean diameter $\geq$ 18 mm. Daily administration of gonadotrophins and the GnRH agonist was discontinued on the day of hCG administration. Transvaginal oocyte retrieval was scheduled 36 hours later. Serum E2 and P levels were measured on the morning of hCG administration. Samples were tested with a microparticle enzyme immunoassay Axsym System (Abbott Cientifico S.A., Madrid, Spain). The serum E2 kit had a sensitivity of 28 pg/mL and intraobserver and interobserver variation coefficients of 6.6% and 7.7%, respectively.

**Protocol for endometrial preparation of recipients**: can be found in Meseguer, M. 2008; Meseguer, M. 2010. Briefly, patients with ovarian function were downregulated with a single dose of 3.75 mg of Triptorelin (Decapeptyl 3.75, Ipsen Pharma S.A., Madrid,

Spain) administered IM in the secretory phase of the previous cycle. Hormonal replacement started on day 1 of the cycle after ovarian downregulation was confirmed with vaginal ultrasound. Patients started oral administration of 2 mg/day of E$_2$ valerate (Progynova, Schering Spain, Madrid, Spain) from days 1 to 8; 4 mg/day from days 9 to 11; and 6 mg/day from day 12 on. Patients without ovarian function started hormonal replacement directly. After 14 days of E$_2$ valerate administration, vaginal ultrasound was performed and serum E$_2$ determined. If recipients were ready to receive oocytes, they waited having 6 mg/day of E$_2$ valerate until an adequate donation was available.

After embryo transfer for luteal phase support all patients received a daily dose of 200 mg for standard patients and 400mg for oocyte recipients of vaginal micronized progesterone (Progeffik Effik, Madrid Spain) every 12 hours.

*Ovum pick-up and ICSI*

Follicles were aspirated and the oocytes were washed in Quinn's Advantage medium (QAM) (SAGE, Rome, Italy). After washing, oocytes were cultured in Quinn's Advantage Fertilization medium (QAFM) (Sage Rome, Italy) at 5.2% CO2 and 37°C for 4 hours before oocyte denudation. Oocyte stripping was carried out by mechanical pipetting in 40IU/mL of hyaluronidase in the same medium (QAFM). After this ICSI was performed in a medium containing HEPES (QAM) (Garcia-Herrero,S. 2010). ICSI was performed at 400x magnification using an Olympus IX7 microscope. Finally the oocytes was placed in pre-equilibrated slides (EmbryoSlide® Unisense FertiliTech, Aarhus Denmark).

*Incubation*

The slides are constructed with a central depression containing 12 straight-sided cylindrical wells each containing a culture media droplet of 20 $\mu$L Quinn's Advantage Cleavage medium (QACM). The depression containing the 12 wells was filled with an overlay of 1.4 mL mineral oil to prevent evaporation. The slides were prepared at least 4 hrs in advance and left in an incubator to pre-equilibrate at 37°C in the 5.0 % CO2 atmosphere. After pre-equilibration all air bubbles are meticulously removed before the oocytes are placed individually in dropplets and incubated in the time-lapse monitoring system until embryo transfer 72 hour later approximately. The time-lapse instrument, EmbryoScope®, (ES), (Unisense FertiliTech, Aarhus, Denmark) is a tri gas oocyte/embryo incubator with a built in microscope to automatically acquire images of up to 72 individual embryos during development.

*Imaging system*

The imaging system in the ES uses low intensity red light (635 nm) from a single LED with short illumination times of 30 ms per image to minimize embryo exposure to light and to avoid damaging short wavelength light. The optics comprise of a modified Hoffmann contrast with a 20x speciality objective (Leica Place) to provide optimal light sensitivity and resolution for the red wavelength. The digital images are collected by a highly sensitive CCD camera (1280 x 1024 pixels) with a resolution of 3 pixels per $\mu$m. Image stacks were acquired at 5 equidistant focal planes every 15 minutes during embryo development inside the ES (i.e. from about 1 hr after fertilization to transfer on day 3 about 72 hrs after fertilization). Embryo exposure to light during incubation was measured with a scalar irradiance microsensor with a tip diameter of 100 $\mu$m placed within the EmbryoScope at the position of the embryo in the EmbryoSlide. Similar measurements were made on standard microscopes used in fertility clinics. The total exposure time in the time-lapse system during 3 day culture and acquisition of 1420 images were 57 seconds, which compares favourably with the 167s microscope light exposure time reported for a standard IVF treatment (Ottosen et al 2007). As the light intensity measured with the within the ES with the scalar irradiance microsensor was much lower than the light intensity in microscopes used in IVF clinics, the total light dose during 3 day incubation in the time-lapse system was found to be 20 J/m2 (i.e. 0.24 $\mu$J/embryo) as opposed to an exposure of 394 J/m2 during microscopy in normal IVF treatments (i.e. 4.8 $\mu$J/embryo) based on average illumination times from (Ottosen et al 2007) and measured average intensities with the scalar irradiance microsensor. Furthermore, the spectral composition of the light in the ES was confined to a narrow range centred around 635 nm, and thus devoid of low wavelength light below 550 nm, and comprise about 15% of the light encountered in a normal IVF microscope.

*Embryo score and culture conditions*

Successful fertilization was assessed at 16-19 h post-ICSI based on digital images acquired with the time-lapse monitoring system. Embryo morphology was evaluated on days 2 (48 h post ICSI) and 3 (72 h post ICSI) based on the acquired digital images, taking into account the number, symmetry and granularity of the blastomeres, type and percentage of fragmentation, presence of multinucleated blastomeres and degree of compaction as previously described (Alikani, M. 2000). Embryo selection were performed exclusively by morphology based on: i) absence of multinucleated cells; ii) between 2-5 cells on day-2; iii) between 6-10 cells on day 3; iv) total fragment volume of less than 15% of the embryo and; v) the embryo must appear symmetric with only slightly asymmetric blastomeres (Meseguer, M. 2006; Muriel, L. 2006; Meseguer, M.
2008). A total of 522 embryos were transferred to 285 patients.

*Time-lapse evaluation of morphokinetic parameters*

Retrospective analysis of the acquired images of each embryo was made with an external computer, EmbryoViewer ® workstation (EV), (Unisense FertiliTech, Aarhus , Denmark) using image analysis software in which all the considered embryo developmental events were annotated together with the corresponding timing of the events in hrs after ICSI microinjection. Subsequently the EV was used to identify the precise timing of the 1st cell division. This division was the division to 2 cells and a shorthand notation of t2 is used in the following. Annotation of the 2nd (i.e. to 3 cells, t3), 3rd (4 cells, t4) and 4th (5 cells, t5) cell division were done likewise. For the purpose of this study, time of cleavage was defined as the first observed timepoint when the newly formed blastomeres are completely separated by confluent cell membranes. All events are expressed as hours post ICSI microinjection.
The duration of the second cell cycle was defined as the time from division to a two blastomere embryo until division to a 3 blastomere embryo. cc2=t3-t2, i.e. the second cell cycle is the duration of the period as 2 blastomere embryo.
The second synchrony s2 was defined as the duration of the division from a 2 blastomere embryo to a 4 blastomere embryo (s2=t4-t3) which corresponds to the duration of the period as 3 blastomere embryo.
The detailed analysis was performed on transferred embryos with 100% implantation (i.e. where the number of gestational sacs confirmed by ultrasound matched the number of transferred embryos) (N=61 and on embryos with 0% implantation, (where no biochemical pregnancy was achieved) (N=186).

*Embryo transfer*

The number of embryos transferred was normally two, but in some cases 1 or 3 embryos were transferred because of embryo quality or patient wishes. Supernumerary embryos were frozen for potential future transfers using IVI standard vitrification technique (Cobo et al. 2008). The $\beta$-hCG value was determined 13 days after embryo transfer and the clinical pregnancy was confirmed when a gestational sac with foetal heartbeat was visible after 7 weeks of pregnancy.

*Statistical Analysis*

The exact timings of embryo events in hrs after ICSI microinjection largely followed normal distributions for the implanted embryos, but that was typically not the case for the not implanted embryos (Shapiro-Wilk test). The distributions of the not implanted embryos typically had long tails extending to later timing values. To investigate whether the variances in the exact timings of embryo events were different between the implanted and not implanted embryos the Brown-Forsythe's test for homogeneity of variances was used, since it does not demand normality of the tested distributions. The Mann-Whitney U-test was used to test whether the median values in the exact timings of embryo events were significantly different between the implanted and not implanted embryos.

To describe the distribution of the probabilities of implantation, timings were converted from continuous variables into a categorical variable using quartiles for all observations of each of the measured parameters. A system based on ordinations giving four categories (timing quartiles) with equal number of observations in each of them was used to obtain these categories. By this procedure, bias due to differences in the total number of embryos in each category was avoided. Hereafter the percentage of embryos that implanted for each timing quartile was calculated to assess the distribution of implantation in the different categories.

The derived embryo timings were analyzed using Student's T-test when comparing two groups, and Analysis of Variance (ANOVA) followed by Bonferroni's and Scheffe's post hoc analysis when multiple groups were considered. Chi square tests were used to compare between categorical data. For each timing variable an optimal range was defined as the combined range spanned by the two quartiles with the highest implantation rates. Additionally, a binary variable was defined with the value inside (outside) if the value of the timing variable was inside (outside) the optimal range.

The odds ratio (OR) of the effect of all binary variables generated on implantation were expressed in terms of 95 % confidence interval (CI95) and significance. By performing the logistic regression analysis, the effect of optimal ranges and other binary variables on implantation were quantified. Significance was calculated using the omnibus test (likehood ratio), and the uncertainties uncovered by the model were evaluated by Negelkerke $R^2$, a coefficient that is analogous to the $R^2$ index of the linear regression analysis. ROC curves were employed to test the predictive value of all the variables included in the model with respect to implantation. ROC curve analysis provides AUC values (area under the curve) that are comprised between 0.5 and 1 and can be interpreted as a measurement of the global classification ability of the model.

Statistical analysis was performed using the Statistical Package for the Social Sciences 17 (SPSS Inc., Chicago, IL) and MedCalc Software (Ghent, Belgium).

**Results**

The primary etiology of female infertility was: poor oocyte quality 34.7% (n=99); advanced maternal age 24.6% (n=70); premature ovary failure 6.0% (n=17); normal 23.8% (n=68), tubal obstruction 2.5% (n=7); low ovary response 8.4% (n=24). Average $E_2$ levels prior to hCG injection were 1701 (SD=991) pg/ml. A total of 201 embryos gave successful implantation (gestational sac) out of the total 522 transferred, giving rise to a 38.5% implantation rate. The biochemical pregnancy rate per transfer was 55.1% (n=157) and ongoing pregnancy rate per transfer were 49.8% (n=142).

A single gestational sac was frequently observed after dual embryo transfer. As it was not possible to ascertain with certainty, which of the two transferred embryos that implanted, these embryos were excluded from further analysis. All embryos with known implantation were selected for further retrospective analysis. This analysis comprise 247 embryos; 61 with 100% implantation (number of gestational sacs matched with number of transferred embryos) and 186 with 0% implantation (no biochemical pregnancy was achieved).

*Time-lapse based morphokinetic parameters and implantation rate*

The correlation between morphokinetic parameters analyzed with the EV time lapse tool and embryo implantation was investigated. For 51 embryos of the total 247 (20.6%) morphological events were observed that were apparently related to poor embryo development. These three events were; A) Direct cleavage from zygote to 3 blastomere embryo, defined as: cc2 = t3 - t2 < 5 hours. (N=9). B) Uneven blastomere size at the 2 cell stage during the interphase where the nuclei are visible (N=26). Blastomeres are considered uneven sized if the average diameter of the large blastomere is more than 25% larger than the average diameter of the small blastomere. This definition implies that the volume of the large blastomere should be at least twice the volume of the small blastomere. C) Multinucleation at the 4 cell stage during the interphase where the nuclei are visible (N=28) The embryo is considered multinucleated if more than one distinct nucleus is observed in one (or more) blastomeres,. From those 51 embryos only 4 implanted (8%) (two with uneven blastomere size and two that were multinucleated). Given the low implantation rate observed in the embryos showing these events it was suggested to use the listed observations as exclusion criteria for embryo selection as the frequency of implanting was very low (4 out of 51 i.e. 8%).

*Timing of embryo development events and implantation*

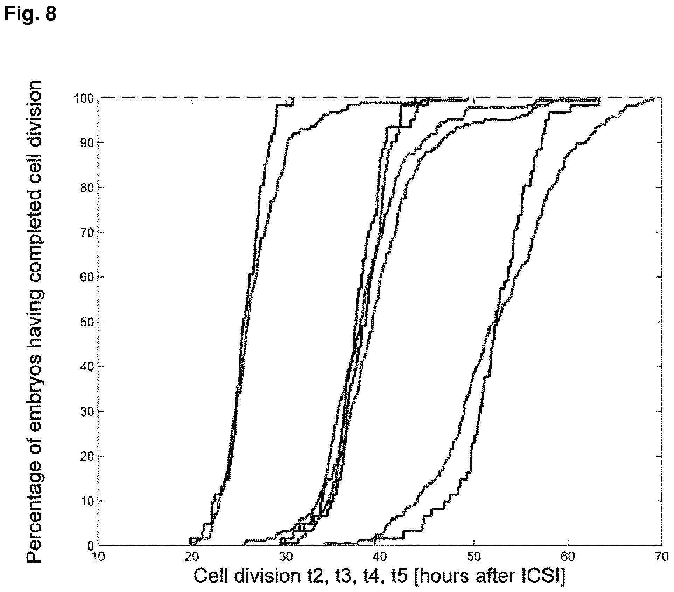

Cleavage times for the first four divisions are shown in Figure 8 as percentages of embryos that have completed their cell division at different time-points after fertilization by ICSI. The four blue curves represent the successive divisions of the 61 embryos, which implanted and the four red curves the 186 embryos that did not.
It is apparent that there is a tighter distribution of cleavage times for implanting embryos as opposed to non-implanting embryos. A prominent tail of lagging embryos was found for the non-implanting embryos (red curves). At least for the late cleavages there also appeared a leading tail of too early cleaving embryos that were found not to implant.
More detailed evaluation of the distribution of all divisional timings was performed. An example, the timing for cell division to five cells, $t_5$, is shown in Figure 9. The distribution of cleavage times for 61 implanting embryos (positive) are indicated by blue dots and for 186 non-implanting embryos (negative) by red dots. The left panel show the overall distributions of cleavage times for the respective embryo types. The right panel show the distribution of observed $t_5$ cleavage times for the two embryos types plotted on a normal quantile plot. Observations following a normal distribution will fall along a straight line on this type of plot. As is evident from the two fitted lines, the cleavage time, $t_5$, appears to follow a normal distribution for both types of embryos. The fitted lines intersect at 0.5, which implies that the mean value of $t_5$ is similar for both groups, but the slopes of the lines differ, indicating that the standard deviations for the two types of embryos are not the same. The slope of the positive (implanting) group is more horizontal and the variance thus expected to be significantly lower for $t_5$ from implanting embryos.

**Table 1.**

| Paramete r | All embryos | | | Implanted embryos | | | | Not implanted embryos | | | | Homogeneit y of variances |
|---|---|---|---|---|---|---|---|---|---|---|---|---|
| | Mean [h] | SD [h] | N [#] | Mean [h] | SD [h] | N [#] | Normal dist. | Mean [h] | SD [h] | N [#] | Normal dist. | p-value |
| **t2** | 26.4 | 3.5 | 247 | 25.6 | 2.2 | 61 | yes | 26.7 | 3.8 | 186 | no | 0.022 |
| **t3** | 38.2 | 4.7 | 246 | 37.4 | 2.8 | 61 | yes | 38.4 | 5.2 | 185 | no | 0.002 |
| **t4** | 39.5 | 5.0 | 243 | 38.2 | 3.0 | 61 | yes | 40.0 | 5.4 | 182 | no | 0.004 |
| **t5** | 52.6 | 6.2 | 228 | 52.3 | 4.2 | 61 | yes | 52.6 | 6.8 | 167 | yes | <0.001 |
| **cc2** | 11.8 | 2.9 | 246 | 11.8 | 1.2 | 61 | yes | 11.8 | 3.3 | 185 | no | 0.006 |
| **s2** | 1.52 | 2.51 | 243 | 0.78 | 0.73 | 61 | no | 1.77 | 2.83 | 182 | no | 0.016 |

EP 2 811 016 B1

The average timing of t2, t3, t4 and t5, together with cc2 and s2 for the analysed transferred embryos with known outcome are presented in Table 1, values for those implanted and not implanted were also calculated. The standard deviation for each of the variables is also included in the table. Additionally, the results of the Shapiro-Wilk test for normal distribution are included in Table I. Exact timings of embryo events follow normal distributions for the implanted embryos for all parameters (except s2). On the other hand the exact timings of embryo events for the not implanted embryos don't follow normal distributions, but exhibit tails at the later timings. Only the parameter t5 follows a normal distribution for the not implanted embryos (see also Figure 9).

As expected from the distributions of cleavage times shown in Figure 8, all the distributions of parameters from implanted embryos are characterized by significantly smaller variances than the distributions of parameters from the non-implanting embryos (Brown-Forsythe's test for homogeneity of variances, p-values shown in Table 1).

This supports the hypothesis that viable embryos follow a predefined developmental schedule with greater fidelity than non-implanting embryos.

Since all parameters show significantly different variances the non-parametric Mann-Whitney U-test was used for comparison of the medians. The median values were not significantly different between the implanting and not implanting embryos for any of the parameters except for s2. The s2, synchrony of second and third cell division were significantly different between implanted embryos with median value 0.50h and not-implanted embryos with median value 1.00h, (p=0.0040).

**Table 2.**

| Parameter | Q1 | | Q2 | | Q3 | | Q4 | |
|---|---|---|---|---|---|---|---|---|
| | Limit [h] | Implantation [%] | Limit [h] | Implantation [%] | Limit [h] | Implantation [%] | Limit [h] | Implantation [%] |
| **t2** | <24.3 | 23 | **24.3-25.8** | **32** | **25.8-27.9** | **30** | >27.9 | 15 |
| **t3** | <35.4 | 18 | **35.4-37.8** | **39** | **37.8-40.3** | **32** | >40.3 | 11 |
| **t4** | < 36.4 | 23 | **36.4-38.9** | **36** | **38.9-41.6** | **31** | >41.6 | 10 |
| **t5** | <48.8 | 16 | **48.8-52.3** | **37** | **52.3-56.6** | **40** | >56.6 | 14 |
| **cc2** | **<11.0** | **23** | **11.0-11.9** | **39** | 11.9-12.9 | 18 | >12.9 | 19 |
| **s2** | **<0.30** | **36** | **0.30-0.76** | **28** | 0.76-1.50 | 20 | >1.50 | 16 |

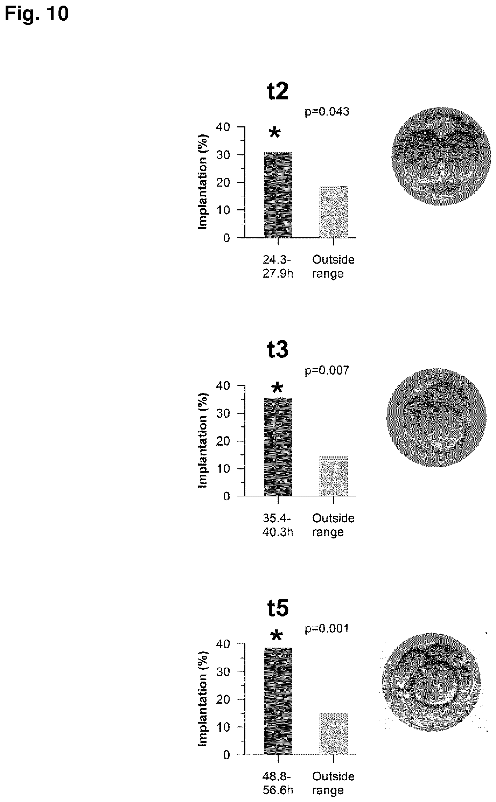

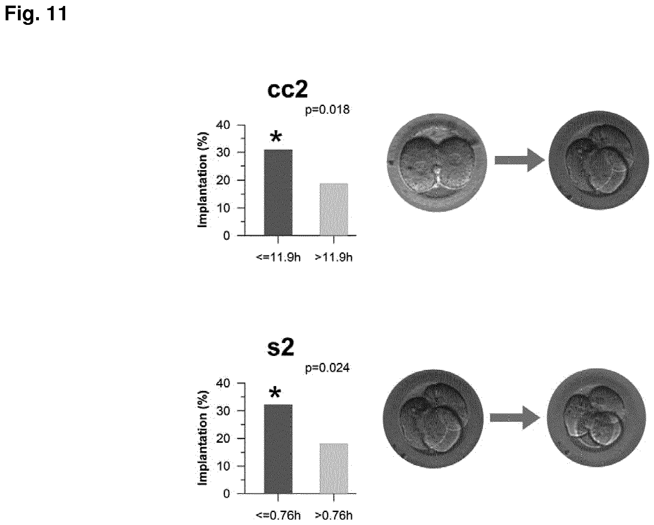

The four quartiles for the timing of each of the investigated parameters are presented in Table 2 together with percentages of implanting embryos in each quartile. The categories defined by these quartiles were used to establish optimal ranges based on the two consecutive quartiles with highest implantation probabilities (entries in bold typeface in Table 2). Observed parameters with significantly higher implantation rate for parameters inside the optimal range as compared to outside the range are presented in Figure 10 and Figure 11.

For all cleavage times assessed (t2, t3, t4 and t5), embryos whose cleavage was completed in the two central quartiles displayed the highest implantation rates, and were consequently combined in an optimal range for each parameter (Figure 10). The finding that the implantation rate in the first quartile for these cleavage times was lower than the two subsequent quartiles indicate that there may be a disadvantage of "too early cleavage". This effect would not have been visible if cleavage timings of all embryos in the investigated IVF cycles had been included, but because the analysis were restricted to only good quality transferred embryos from these cycles, a lower limit for the optimal cleavage range for t2, t3, t4 and t5 could be determined.

For all cleavage times there was a significant difference in implantation rate between embryos within the optimal range as opposed to those outside the range (Figure 10). However, it should be noted that the discrimination between implantation rates within the two best quartiles and the implantation rate outside these quartiles increased with successive cell divisions. For t2 the difference in implantation rate was 12%, for t3 a difference on 21% was found, and for t5 it amounted to 24%. The implantation of embryos with t5 cleavage within the range was 2.6x the implantation rate for embryos outside this range. Selection based on the timing of cleavage to the 5-cell stage thus provided the best single criteria to select embryos with improved implantation potential.

For both the duration of the second cell cycle, cc2, and the synchrony of cell cleavages in the transition from 2-cell stage to 4-cell stage, s2 (i.e. the duration of the three cell stage), the embryos that cleaved in the two first quartiles was found to have significantly higher implantation rate that those falling in the last two quartiles (Figure 11). Eliminating from this analysis the embryos where abrupt cell division from one cell to three or four cells were observed, i.e. embryos where cc2 < 5 hrs, the implantation rate in the first quartile for cc2 would be higher (26% instead of 23%). Such abnormal divisions were rare and only seen in 9 of the 247 investigated embryos, none of these embryos implanted

*Evaluation of potential selection parameters based on a logistic regression analysis*

A logistic regression analysis were used to select and organize which observed timing events, expressed as binary variables inside or outside the optimal range as defined above, should be used together with the morphological exclusion criteria. The model identified the time of division to five cells, t5 OR=3.31 (CI95% 1.65-6.66) followed by synchrony of divisions after the two cell stage, s2 OR=2.04 (CI95% 1.07-4.07) and the duration of the second cell cycle, cc2 OR=1.84 (CI95% 0.95-3.58) as the most promising variables characterizing implanting embryos.

A logistic regression model was defined by using exclusion variables plus t5, s2 and cc2. A ROC curve analysis to determine the predictive properties of this model with respect to probability of implantation gave an area under the curve AUC value of 0.720 (CI95% 0.645-0.795).

These data was used to generate the hierarchical selection model described herein (Fig. 2 and 3).

## Example 2 - data analysis based on known implantation data

This analysis is based on known implantation data (KID) of 1598 embryos from 10 different clinics. The KID embryos are all transferred embryos with known implantation. With multiple embryos were transferred only total failure of implantation or total success is used. All multiple transfers with implantation that have less implanted embryos than transferred were discarded to enable the implantation success for the specific embryo. The implantation success takes the value 1 if the transferred embryo implanted and 0 if not. The number of embryos (N) used for calculating the expected value (probability of success) of the target and non-target groups is different for different variables.

*Single variable*

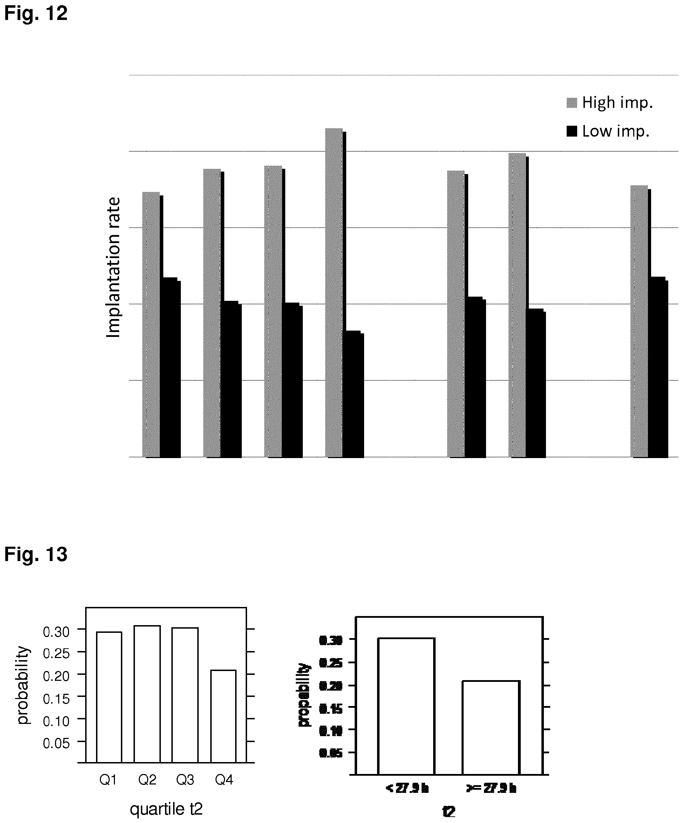

The data were divided into quartiles with respect to a single continuous variable (e.g. t2) and the expected value (probability of getting a success with one trial) of each quartile was calculated. From these quartile groups a new group was formed (the target group) either by the quartile with the highest expected value or by two or three neighboring quartiles having similar probability (see example in figure 13). A Fisher's exact test was used to test the hypothesis that the probability of implanting (expected value of the KID data) of embryos in the target group and outside the group was equal (Table 3). The hypothesis was rejected if the p-value was < 0.1 indicating that there was a difference between groups, and otherwise considered non-significant.

Table 3: Statistical evaluation of the probability of embryos implanting using single continuous time-lapse variables for all annotated embryos

| Variable | Target group quartiles | Target group interval | Probability Inside/outside target group | N Inside/outside Target group | Fisher test p-value |
|---|---|---|---|---|---|
| | | **All** | **0.28** | **1598** | |
| t2 | Q1, Q2, Q3 | < 27.9 h | 0.30/0.21 | 1198/400 | 0.0003 |
| t5 | Q2, Q3 | 48.7 h - 55.6 h | 0.31/0.26 | 779/779 | 0.02 |
| t8 | Q1, Q2 | < 57.2 h | 0.34/0.29 | 604/607 | 0.09 |
| cc2 (t3-t2) | Q1, Q2, Q3 | < 12.7 h | 0.30/0.22 | 1177/421 | 0.004 |
| cc2b (t4-t2) | Q1, Q2 | < 12.7 h | 0.33/0.23 | 797/798 | <0.0001 |
| cc3 (t5-t3) | Q2, Q3 | 12.9 h - 16.3 h | 0.31/0.26 | 780/778 | 0.02 |
| s2 (t4-t3) | Q1 | < 0.33 h | 0.32/0.27 | 368/1227 | 0.06 |
| s2 (t4-t3) | Q1, Q2, Q3 | < 1.33 h | 0.30/0.23 | 1195/400 | 0.004 |
| s3 (t8-t5) | Q1 | < 2.7 h | 0.38/0.29 | 298/913 | 0.009 |

(continued)

| Variable | Target group quartiles | Target group interval | Probability Inside/outside target group | N Inside/outside Target group | Fisher test p-value |
|---|---|---|---|---|---|
| cc2_3 (t5-t2) | Q2, Q3 | 24.0 h- 28.7 h | 0.32/0.25 | 778/780 | 0.002 |

Table 4: Statistical evaluation of the probability of embryos implanting using discrete variables for all annotated embryos. The probability for implantation of the whole dataset was 0.28 with 1598 observations (N).

| Variable / target group | Probability Inside/outside target group | N Inside/outside Target group | Fisher test p-value |
|---|---|---|---|
| **The whole dataset** | **0.28** | **1598** | |
| No multinuclation at t2 | 0.31/0.23 | 984/608 | 0.0007 |
| No Multinucleation at t4 | 0.31/0,18 | 1238/241 | <0.0001 |
| Even blastocycts at t2 | 0.28/0.18 | 1509/90 | 0.03 |
| t8 observed in less than 60 h | 0.33/0.23 | 781/817 | <0.0001 |
| t7 observed in less than 60 h | 0.32/0.19 | 1086/512 | <0.0001 |
| t6 observed in less than 60 h | 0.30/0.18 | 1305/293 | <0.0001 |
| cc2 is more than 5 h | 0.29/0.03 | 1531/67 | <0.0001 |
| cc3 is more than 5 h | 0.29/0.18 | 1475/83 | <0.03 |
| cc1 (t2) is less than 32 h | 0.29/0.06 | 1515/83 | <0.0001 |

All the variables tested in table 4 can be used to exclude embryos with very low implantation rate since the implantation success rates of the embryos outside the target groups are 0.23 and below. The two criteria cc2 < 5 h and cc3 < 5 h are associated with a low implantation success rate. This may be due to direct cleavage from 1 to 3 blastomeres and 2 to 5 blastomeres indicating a mismatch in DNA replication or in the cell division in general. The embryos with these irregular division patterns will have asynchronous time-lapse data and may disturb any statistical calculation if they are included in the data. The embryos with cc1 (t2) longer than 32 h are also associated with a low implantation success rate and are embryos that develop slowly, possibly due to immaturity of the oocytes.

*Composite variables*

Another option is to use composite variables calculated using the primary morpho-kinetic variables (timings and time periods). Especially interesting are variables that express the ratio between two morphological time periods. These types of normalized variables may hold information that is better for predictive models since they may take out some of the variability that may arise due to differences in temperature and other environmental variables and since they may be less sensitive to the definition of fertilization time. This could for example be cc2/cc2_3 and cc3/cc2_3 (the fraction of the first and second cell cycle out of the first two cell cycles) or s2/cc2 and s3/cc3 (the synchronicity of the first cell or second cell cycle relative to the time of the first or second cell cycle). The timing of the individual cell divisions in s3 (t8-t5), i.e. s3a (t6-t5), s3b (t7-t6), s3c (t8-t7) is believed to be of interest since they may demonstrate the competence of each individual cell to perform a cell division. Possible irregularities or abnormalities in the mitosis may result in large differences between the value of s3a, s3b and/or s3c (i.e. one high max value).

Table 4: Quartile analysis of the composite variables, the target group, the implantation success rate (probability) for the embryos inside and outside the target groups, the number of observations in the target and non-target group and the p-value for Fisher's exact test.

| Variable | | Target group | Probability Inside/outside target group | N Inside/outside Target group | p-value Fishers test |
|---|---|---|---|---|---|
| | | | | | |
| $cc2/cc2\_3=(t3-t2)/(t5-t2)$ | Q2, Q3 | 0.42 - 0.47 | 0.32/0.25 | 779/779 | 0.002 |
| $cc3/cc2\_3=(t5-t3)/(t5-t2)$ | Q2, Q3 | 0.53 - 0.58 | 0.32/0.25 | 779/779 | 0.002 |
| $cc3/t5 = 1-t3/t5$ | Q1,Q2,Q3 | < 0.30 | 0.30/0.23 | 1181/395 | 0.012 |
| $cc3/t5 = 1-t3/t5$ | Q2 | 0.26 - 0.28 | 0.32/0.23 | 395/1181 | 0.06 |
| $s2/cc2 = (t4-t3)/(t3-t2)$ | Q1 | < 0.025 | 0.32/0.27 | 797/798 | 0.05 |
| $s3/cc3 = (t8-t5)/(t5-t3)$ | Q1 | < 0.18 | 0.36/0.30 | 302/909 | 0.07 |
| $cc2/cc3 = (t3-t2)/(t5-t3)$ | Q2, Q3 | 0.72 - 0.88 | 0.32/0.25 | 779/779 | 0.0016 |
| Max(s3a, s3b, s3c) | Q1 | < 1.5 h | 0.36/0.30 | 279/932 | 0.06 |
| Max(s3a, s3b, s3c)/s3 | Q1 | < 0.5 | 0.36/0.30 | 302/907 | 0.07 |

## References

Alikani, M., Calderon, G., Tomkin, G., Garrisi, J., Kokot, M. and Cohen, J. (2000) "Cleavage anomalies in early human embryos and survival after prolonged culture in-vitro" Hum Reprod, vol. 15 (12), pp. 2634-2643

Arav, A., Aroyo, A., Yavin, S. and Roth, Z. (2008) "Prediction of embryonic developmental competence by time-lapse observation and 'shortest-half' analysis" Reprod Biomed Online, vol. 17 (5), pp. 669-675

Bos-Mikich, A., Mattos, A.L.G. and Ferrari, A.N. (2001) "Early cleavage of human embryos: an effective method for predicting successful IVF/ICSI outcome" Hum Reprod, vol. 16 (12), pp. 2658-2661

Brezinova, J., Oborna, I., Svobodova, M. and Fingerova, H. (2009) "Evaluation of day one embryo quality and IVF outcome - a comparison of two scoring systems" Reprod Biol Endocrinol, vol 7. (9), doi:10.1186/1477-7827-7-9

Ciray HN, Karagenc L, Ulug U, Bener F, Bahceci M. Use of both early cleavage and day 2 mononucleation to predict embryos with high implantation potential in intracytoplasmic sperm injection cycles. Fertil Steril 2005;84:1411-6.

Ciray HN, Karagenc L, Ulug U, Bener F, Bahceci M. Early cleavage morphology affects the quality and implantation potential of day 3 embryos. Fertil Steril 2006;85:358-65.

Cruz, M., Gadea, B., Garrido, N., Pedersen, K.S., Martinez, M., Perez-Cano, I., Muñoz, M. and Meseguer, M. (2011) "Embryo quality, blastocyst and ongoing pregnancy rates in oocyte donation patients whose embryos were monitored by time-lapse imaging" J Assist Reprod Genet, in press, Available online DOI 10.1007/s10815-011-9549-1

Cruz, M., Garrido, N., Perez-Cano, I., Muñoz, M., Pellicer, A., Martinez, M., Gadea, B., Sellés, E., Betersen, J. and Meseguer, M. (2010) "Time lapse video analysis provide precise division kinetics of cultured embryos which corellates with blastocyst formation and quality" Abstracts of the 26th Annual Meeting of ESHRE, Rome, Italy, p. i203

Cruz, M., Nicolás, G., Inmaculada, P.-C. , R. Niels, M. Manuel, M. Marcos (2010) "Comparative study of embryo quality, blastocyst and ongoing pregnancy rates in oocyte donation patients sharing embryoscope and standard incubator." Fertil Steril, vol. 94 (4) (Suppl), p. S78

De los Santos, M. J., Escrich, L., Grau, N., Pellicer, A., Romero, J.L. and Escribá, M.J. (2010) "Development of tripronucleated ICSI-derived embryos using real time assessment" Abstracts of the 26th Annual Meeting of ESHRE, Rome, Italy, p. i192

Elliott, T., Kahn, J., Lowderman, J., Wright, G., Chang, C., Bernal, D., Kort, H. and Nagy, Z. (2010) "Time-lapse video evidence of murine embryonic developmental regression in sub-optimal culture conditions using Embryoscope™" Abstracts of the 26th Annual Meeting of ESHRE, Rome, Italy, p. i192

Escrich, L., Grau, N., Garcia-Bautista, A., Galán, A., Pellicer, A. and Escribá, M.J. (2010)"Real-time evaluation of the different stages of meiosis during spontaneous in vitro maturation of human GV oocytes." Fertil Steril, vol. 94 (4) (Suppl), p. S141

Fancsovits, P., Takács, F. Z., Tóthné, G. Z., Papp, Z. and Urbancsek, J. (2006) "Examination of Early Cleavage and its Importance in IVF Treatment" J Reproduktionsmed Endokrinol, vol. 3 (6), pp. 367-372

Fawcett, T (2006) "An introduction to ROC analysis" Pattern Recognition Letters, vol 27, pp. 861-874.

Fenwick, J., Platteau, P., Murdoch, A.P. and Herbert, M. (2002) "Time from insemination to first cleavage predicts developmental competence of human preimplantation embryos in vitro" Hum Reprod, vol. 17 (2), pp. 407-412

Finn, A., Scott, L., O'Leary, T., Davies, D. and Hill, J. (2010) "Sequential embryo scoring as a predictor of aneuploidy in poor-prognosis patients" Reprod Biomed Online, vol. 21, pp. 381-390

Giorgetti, C., Hans, E., Terriou, P., Salzmann, J., Barry, B., Chabert-Orsini, V., Chinchole, J.M., Franquebalme, J.P., Glowaczower, E., Sitri, M.C., Thibault, M.C. & Roulier, R. 2007, "Early cleavage: an additional predictor of high implantation rate following elective single embryo transfer", Reproductive biomedicine online, vol. 14, no. 1, pp. 85-91.

Grau, N., Escrich, L., Ramsing, N., Garcia-Bautista, A., Pellicer, A. and Escribá, M.J. (2010) "A new approach to determining the optimal duration of MII arrest in vitro-matured human GV oocytes" Fertil Steril, vol. 94 (4) (Suppl), p.S141

Grisart, B., Massip, A. and Dessy, F. (1994) "Cinematographic analysis of bovine embryo development in serum-free oviduct-conditioned medium" J Reprod Fertil, vol. 101, pp. 257-264

Hardarson, T., Hanson, C., Sjögren, A. and Lundin, K. (2001) "Human embryos with unevenly sized blastomeres have lower pregnancy and implantation rates: indications for aneuploidy and multinucleation." Hum Reprod, vol. 16 (2), pp. 313-318

Hardarson, T., Löfman, C., Coull, G., Sjögren, A., Hamberger, L. and Edwards, R.G. (2002) "Internalization of cellular fragments in a human embryo: time-lapse recordings." Reprod Biomed Online, vol. 5(1), pp. 36-38

Herrero, J., Alberto, T., Ramsing, N. B., De los Santos, M. J., Escrich, L. and Meseguer, M. (2010) "Linking successful implantation with the exact timing of cell division events obtained by time-lapse system in the embryoscope." Fertil Steril, vol. 94 (4) (Suppl), p. S149

Hesters, L., Prisant, N., Fanchin, R., Méndez Lozano, D. H., Feyereisen, E., Frydman, R., Tachdjian, G. and Frydman, N. (2008) "Impact of early cleaved zygote morphology on embryo development and in vitro fertilization-embryo transfer outcome: a prospective study" Fertil Steril, vol. 89 (6), pp.1677-1684

Holm, P. Shukri, N.N., Vajta, G., Booth, P., Bendixen, C. and Callesen, H. (1998) "Developmental kinetics of the first cell cycles of bovine in vitro produced embryos in relation to their in vitro viability and sex." Therioge-nology, vol. 50, pp. 1285-1299

Holm, P., Booth, P.J. and Callesen, H. (2002) "Kinetics of early in vitro development of bovine in vivo- and in vitro-derived zygotes produced and/or cultured in chemically defined or serum-containing media." Reprod, vol. 123, pp. 553-565

Holm, P., Booth, P.J. and Callesen, H. (2003) "Developmental Kinetics of Bovine Nuclear Transfer and Parthenogenetic Embryos" Cloning and stem cells, vol. 5 (2), pp. 133-142

Holte, J., Berglund, L., Milton, K., Garello, C., Gennarelli, G., Revelli, A. and Bergh, T. (2007) "Construction of an evidence-based integrated morphology cleavage embryo score for implantation potential of embryos scored and transferred on day 2 after oocyte retrieval." Hum Reprod, vol. 22(2), pp. 548-557

Jones, G.M., Cram, D.S., Song, B., Kokkali, G., Pantos, K. & Trounson, A.O. 2008, "Novel strategy with potential to identify developmentally competent IVF blastocysts", Human reproduction (Oxford, England), vol. 23, no. 8, pp. 1748-1759.

Lechniak, D., Pers-Kamczyc, E. and Pawlak, P. (2008) "Timing of the first zygotic cleavage as a marker of developmental potential of mammalian embryos." Reprod Biol, vol. 8(1), pp. 23-42

Lemmen, J.G., Agerholm, I. and Ziebe, S. (2008) "Kinetic markers of human embryo quality using time-lapse recordings of IVF/ICSI-fertilized oocytes." Reprod Biomed Online, vol. 17(3), pp. 385-391

Lequarre, A.S., Marchandise, J., Moreau, B., Massip, A. and Donnay, I. (2003) "Cell Cycle Duration at the Time of Maternal Zygotic Transition for In vitro Produced Bovine Embryos: Effect of Oxygen Tension and Transcription Inhibition" Biol Reprod, vol. 69, pp. 1707-1713

Lopes, A.S., Greve, T. and Callesen, H. (2007) "Quantification of embryo quality by respirometry" Theriogenology, vol. 67, pp. 21-31

Lopes, A.S., Lane, M. and Thompson, J.G. (2010) "Oxygen consumption and ROS production are increased at the time of fertilization and cell cleavage in bovine zygotes." Hum Reprod, vol.25(11), pp. 2762-2773

Lopes, A.S., Larsen, L.H., Ramsing, N., Lovendahl, P., Raty, M., Peippo, J., Greve, T. and Callesen, H. (2005) "Respiration rates of individual bovine in vitro-produced embryos measured with a novel, non-invasive and highly sensitive microsensor system" Reprod, vol. 130, pp. 669-679

Lopes, A.S., Madsen, S.E., Ramsing, N.B., Lovendahl, P., Greve, T. and Callesen, H. (2007) "Investigation of respiration of individual bovine embryos produced in vivo and in vitro and correlation with viability following transfer." Hum Reprod, vol. 22, pp. 558-566

Lopes, A.S., Wrenzycki, C., Ramsing, N.B., Herrmann, D., Niemann, H., Lovendahl, P., Greve, T. and Callesen, H. (2007) "Respiration rates correlate with mRNA expression of G6PD and GLUT1 genes in individual bovine in vitro-produced blastocysts" Theriogenology, vol. 68, pp. 223-236

Lundin, K. Bergh, C. and Hardarson, T. (2001) "Early embryo cleavage is a strong indicator of embryo quality in human IVF" Hum Reprod (Oxford, England), vol. 16(12), pp. 2652-2657

Magli, M.C., Gianaroli,L., Ferraretti, A.P., Lappi, M., Ruberti, A, and Farfalli,V. (2007) "Embryo morphology and development are dependent on the chromosomal complement" Fertil Steril, vol. 87 (3), pp. 534-541

Majerus, V., Lequarreâ, A.S., Ferguson, E.M., Kaidi, S., Massip, A., Dessy, F. and Donnay, I. (2000) "Characterization of Embryos Derived From Calf Oocytes: Kinetics of Cleavage, Cell Allocation to Inner Cell Mass, and Trophectoderm and Lipid Metabolism" Mol Reprod Dev, vol. 57, pp. 346-352

Mastenbroek, S., Twisk, M., van Echten-Arends, J., Sikkema-Raddatz, B., Korevaar, J.C., Verhoeve, H.R., Vogel, N.E., Arts, E.G., de Vries, J.W., Bossuyt, P.M., Buys, C.H., Heineman, M.J., Repping, S. & van der Veen, F. 2007, "In vitro fertilization with preimplantation genetic screening", The New England journal of medicine, vol. 357, no. 1, pp. 9-17.

Meng, L., Jastromb, N., Alworth, S. and Jain, J. (2009) "Remote Monitoring and Evaluation of Early Human Embryo Developmentby a Robotic-Operated Culture-Imaging System" Fertil Steril, S7

Meseguer, M., Herrero, J., Tejera, A., De los Santos, M. J., Escrich, L., Garrido, N. and Ramsing, N. (2010) "Embryos with too early first cleavage fail to implant; correlation between exact timing of first cleavage and successful implantation" Abstracts of the 26th Annual Meeting of ESHRE, Rome, Italy, pp. i58-i59

Meseguer, M., Hilligsøe, K.M., Pedersen, K.S., Herrero, J., Tejera, A. and Garrido, N. (2010) "Pregnancy rates after incubation in new time-lapse incubator (EmbryoScope) providing detailed information about embryo development compared to incubation in a standard incubator." Fertil Steril, vol. 94 (4) (Suppl), p. S150

Mio Y. Morphological analysis of human em- bryonic development using time-lapse cinematography. J Mamm Ova Res 2006;23:27-36.

Mio, Y. and Maeda, K. (2008) "Time-lapse cinematography of dynamic changes occurring during in vitro development of human embryos." Am J Obstet Gynecol, vol. 199(6), pp. 660.e1-e5

Muñoz, M., Meseguer, M., Cruz, M., Pérez-Cano, I., Pellicer, A., Gadea, B., Martinez, M., Fortuño, S., Gundersen, J. and Garrido, N. (2010) "Morphometric variations and differences in embryo cleavage kinetics associated with fertilization procedure: classic IVF vs. ICSI" Abstracts of the 26th Annual Meeting of ESHRE, Rome, Italy, pp. i202-i203

Nakahara, T., Iwase, A., Goto, M., Harata, T., Suzuki, M., Ienaga, M., Kobayashi, H., Takikawa, S., Manabe, S., Kikkawa, F. and Ando, H. (2010) "Evaluation of the safety of time-lapse observations for human embryos." J Assist Reprod Genet, vol. 27(2-3), pp. 93-96

Neuber, E., Rinaudo, P., Trimarchi, J.R. and Sakkas, D. (2003) "Sequential assessment of individually cultured human embryos as an indicator of subsequent good quality blastocyst development" Hum Reprod, vol. 18 (6), pp. 1307-1312

Oh, S.J., Gong, S.P., Lee, S.T., Lee, E.J. and Lim, J.M. (2007) "Light intensity and wavelength during embryo manipulation are important factors for maintaining viability of preimplantation embryos in vitro" Fertil Steril, vol. 88 (2), pp. 1150-1157

Ottosen, L.D., Hindkjaer, J., and Ingerslev, J. (2007) "Light exposure of the ovum and preimplantation embryo during ART procedures" J Assist Reprod Genet, vol. 24, pp. 99-103

Paternot, G., Devroe, J., Debrock, S., D'Hooghe, T.M. and Spiessens, C. (2009) "Intra- and inter-observer analysis in the morphological assessment of early-stage embryos" Reprod Biol Endocrinol, vol. 7 (105), doi:10.1186/1477-7827-7-105

Payne, D., Flaherty, S.P., Barry, M.F. and Matthews, C.D. (1997) "Preliminary observations on polar body extrusion and pronuclear formation in human oocytes using time-lapse video cinematography." Hum Reprod, vol. 12(3), pp. 532-541.

Petersen, C. G., Mauri, A. L., Ferreira, R., Baruffi, R. L. R. and Franco, Jr, J. G. (2001) "Embryo Selection by the First Cleavage Parameter Between 25 and 27 Hours After ICSI" J Assist Reprod Genet, vol. 18 (4), pp. 209-212

Pribenszky, C., Losonczi, E., Molnár, M., Lang, Z., Mátyás, S., Rajczy, K., Molnár, K., Kovács, P., Nagy, P., Conceicao, J. and Vajta, G. (2010) "Prediction of in-vitro developmental competence of early cleavage-stage mouse embryos with compact time-lapse equipment" Reprod Biomed Online, vol. 20,pp. 371- 379

Racowsky, C., Ohno-Machado, L., Kim, J. and Biggers, J.D. (2009) "Is there an advantage in scoring early embryos on more than one day?" Hum Reprod, vol. 24 (9), pp. 2104-2113

Ramsing, N. B. and Callesen, H. (2006) "Detecting timing and duration of cell divisions by automatic image analysis may improve selection of viable embryos" Fertil Steril, vol. 86 (3) (Suppl), p. S189

Ramsing, N. B., Berntsen, J. and Callesen, H. (2007) "Automated detection of cell division and movement in time-lapse images of developing bovine embryos can improve selection of viable embryos" Fertil Steril. vol. 88 (Suppl 1), p. S38

Reed, M.L., Hamic, A., Thompson, D.J. and Caperton, C.L. (2009) "Continuous uninterrupted single medium culture without medium renewal versus sequential media culture: a sibling embryo study" Fertil Steril, vol. 92, pp. 1783-1786

Reichman, D.E., Jackson, K.V. and Racowsky, C. (2010) "Incidence and development of zygotes exhibiting abnormal pronuclear disposition after identification of two pronuclei at the fertilization check." Fertil Steril, vol. 94(3), pp. 965-970

Sakkas, D., Percival, G., D'Arcy, Y., Sharif, K. and Afnan, M. (2001) "Assessment of early cleaving in vitro fertilized human embryos at the 2-cell stage before transfer improves embryo selection" Fertil Steril, vol. 76 (6), pp. 1150-1156

Sakkas, D., Shoukir, Y., Chardonnens, D., Grace Bianchi, P. and Campana, A. (1998) "Early cleavage of human embryos to the two-cell stage after intracytoplasmic sperm injection as an indicator of embryo viability" Hum Reprod, vol. 13, pp. 182-187

Scott, L. 2003, "Pronuclear scoring as a predictor of embryo development", Reproductive biomedicine online, vol. 6, no. 2, pp. 201-214.

Scott, L. and Ramsing, N.B. (2007) "Oocyte and embryo respiration rate measurements in a clinical setting - Potential to improve embryo selection?" The Clinical Embryologist, vol. 10 (2), pp. 5-9

Scott, L., Berntsen, J., Davies, D., Gundersen, J., Hill, J. and Ramsing, N. (2008) "Symposium: Innovative techniques in human embryo viability assessment Human oocyte respiration-rate measurement - potential to improve oocyte and embryo selection?" Reprod BioMed Online, vol. 17 (4), pp. 461-469

Scott, L., Finn, A., Kloos, B. and Davies, D. (2010) "The origin and consequences of Day-2 multinucleation of human Embryos" Abstracts of the 26th Annual Meeting of ESHRE, Rome, Italy, p. i195

Scott, L., Finn, A., O'Leary, T., McLellan, S. and Hill, J. (2007) "Morphologic parameters of early cleavage-stage embryos that correlate with fetal development and delivery: prospective and applied data for increased pregnancy rates" Hum Reprod, vol. 22 (1), pp. 230-240

Seli, E., Vergouw, C.G., Morita, H., Botros, L., Roos, P., Lambalk, C.B., Yamashita, N., Kato, O. & Sakkas, D. 2010, "Noninvasive metabolomic profiling as an adjunct to morphology for noninvasive embryo assessment in women undergoing single embryo transfer", Fertility and sterility, vol. 94, no. 2, pp. 535-542.

Shoukir, Y., Campana, A., Farley, T., and Sakkas, D. (1997) "Early cleavage of in-vitro fertilized human embryos to the 2-cell stage: a novel indicator of embryo quality and viability" Hum Reprod, vol. 12, pp. 1531-1536

Sifer, C., Handelsman, D., Grange, E., Porcher, R., Poncelet, C., Martin-Pont, B., Benzacken, B. & Wolf, J.P. 2009, "An auto-controlled prospective comparison of two embryos culture media (G III series versus ISM) for IVF and ICSI treatments", Journal of assisted reproduction and genetics, vol. 26, no. 11-12, pp. 575-581.

Somfai, T., Inaba, Y., Aikawa, Y., Ohtake, M., Kobayashi, S., Konishi, K. and Imai, K. (2010) "Relationship between the length of cell cycles, cleavage pattern and developmental competence in bovine embryos generated by in vitro fertilization or parthenogenesis" J Reprod Dev, vol. 56(2), pp. 200-207

Takenaka, M., Horiuchi, T. and Yanagimachi, R. (2007) "Effects of light on development of mammalian zygotes" PNAS, vol. 104 (36), pp. 14289-14293

Tejera, A., Herrero, J., Ramsing, N. B., Garrido, N., Grau, N. and Meseguer, M. (2010) "Embryo respiration measurements used for quantification of embryo quality; embryo respiration correlates with ongoing pregnancy and implantation in oocyte donation program." Fertil Steril, vol. 94 (Suppl 4), pp. S67-S68

Tejera, A., Herrero, J., Ramsing, N., Pellicer, A., Garrido, N., Grau, N., De los Santos, M.J. and Meseguer, M. (2010) "Quantification of oocyte quality through respiration patterns correlates oxygen consumption with fertilization and subsequent implantation" Hum Reprod, vol. 25 (Suppl 1), pp. i3-i4, doi: 10.1093/humrep/de.25.s1.3

Terriou, P., Giorgetti, C., Hans, E., Salzmann, J., Charles, O., Cignetti, L., Avon, C. & Roulier, R. 2007, "Relationship between even early cleavage and day 2 embryo score and assessment of their predictive value for pregnancy", Reproductive biomedicine online, vol. 14, no. 3, pp. 294-299.

Turczynski, C.J., Chang, C., Hovis, W., Marino, A. and London, S.L. (1999) Culture of Embryos in an Environment Shielded from Exposure to Electro-Magnetic Fields has no Effect on IVF Outcome" Fertil Steril, vol. 72 (3) Suppl. 1

Ugajin, T., Terada, Y., Hasegawa, H., Velayo, C.L., Nabeshima, H. and Yaegashi, N. (2010) "Aberrant behavior of mouse embryo development after blastomere biopsy as observed through time-lapse cinematography" Fertil Steril, vol. 93 (8), pp. 2723-2728

Vajta, G., Korösi, T., Du, Y., Nakata, K., Ieda, S., Kuwayama, M. & Nagy, Z.P. (2008) "The Well-of-the-Well system: an efficient approach to improve embryo development." Reprod Biomed Online, vol. 17 (1), pp. 73-81

Van Montfoort, A.P.A., Dumoulin, J. C.M., Kester, A.D.M. and Evers, J.L.H. (2004) "Early cleavage is a valuable addition to existing embryo selection parameters: a study using single embryo transfers*" Hum Reprod, vol. 19 (9), pp. 2103-2108

VerMilyea, M.D., Graham, J.R., Richter, K.S., Mottla, G.L. and Tucker, M.J. (2010) "Redefining the mouse embryo assay (mea): novel time-lapse imagery and continuous surveillance vs. 'Snap shot' observation." Fertil Steril, vol. 94 (4) (Suppl), p. S212

Wale, P.L. and Gardner, D.K. (2010) "Time-lapse analysis of mouse embryo development in oxygen Gradients" Reprod BioMed Online, vol. 21 (3), pp. 402-410, doi: 10.1016/j.rbmo.2010.04.028

Weitzman, V.N., Schnee-Riesz, J., Benadiva, C., Nulsen, J., Siano, L. and Maier, D. (2010) "Predictive value of embryo grading for embryos with known outcomes" Fertil Steril, vol. 93 (2), pp. 658-662

Windt, M.-L., Kruger, T.F., Coetzee, K. and Lombard, C.J. (2004) "Comparative analysis of pregnancy rates after the transfer of early dividing embryos versus slower dividing embryos" Hum Reprod, vol. 19 (5), pp. 1155-1162

Wong, C.C., Loewke, K.E., Bossert, N.L., Behr, B., De Jonge, C.J., Baer, T.M. and Reijo Pera, R.A. (2010) "Non-invasive imaging of human embryos before embryonic genome activation predicts development to the blastocyst stage." Nat Biotechnol, vol. 28 (10), pp. 1115-1121

Yakin, K., Balaban, B. and Urman, B.(2005) "Impact of the presence of one or more multinucleated blastomeres on the developmental potential of the embryo to the blastocyst stage." Fertil Steril, vol. 83(1), pp. 243-245

Yamagata, K., Suetsugu, R. and Wakayama, T. (2009) "Long-term, six-dimensional live-cell imaging for the mouse preimplantation embryo that does not affect full-term development." J Reprod Dev, vol. 55, pp. 328-331

Yamazaki, T., Yamagata, K. and Baba, T. (2007) "Time-lapse and retrospective analysis of DNA methylation in mouse preimplantation embryos by live cell imaging" Dev Biol, vol. 304, pp. 409-419

Yang, W.J., Hwu, Y.M., Lee, R.K., Li, S.H., Fleming, S. (2009) "Early-cleavage is a reliable predictor for embryo implantation in the GnRH agonist protocols but not in the GnRH antagonist protocols" Reprod Biol Endocrinol, vol. 7 (20), doi:10.1186/1477-7827-7-20

Zhang, J.Q., Li, X.L., Peng, Y., Guo, X, Heng, B.C. and Tong, G.Q. (2010) "Reduction in exposure of human embryos outside the incubator enhances embryo quality and blastulation rate." Reprod Biomed Online, vol. 20, pp. 510- 515

**Further details of the invention**

The invention will now be described in further details with reference to the following items:

1. A method for determining embryo quality comprising monitoring the embryo for a time period, and determining one or more quality criteria for said embryo, and based on said one or more quality criteria determining the embryo quality.

2. The method according to item 1, wherein the embryo quality is determined from a plurality of said quality criteria, such as by combining a plurality of said quality criteria.

3. The method according to any of the preceding items, wherein the quality criterion is a criterion relating to the phase of from 2 to 8 blastomere embryo, or from 4 to 8 blastomere embryo.

4. The method according to any of the preceding items, wherein the quality criterion is a criterion obtained within the time period of from 48 to 72 hours from fertilisation.

5. The method according to any of the preceding items, wherein a population of embryos is monitored.

6. The method according to any of the preceding items, wherein the embryo quality is a quality relating to implantation success.

7. The method according to any of the preceding items, wherein said one or more quality criteria are combined with one or more exclusion criteria for deselecting and/or excluding embryos with a low probability of implantation success.

8. The method according to item 7, wherein an exclusion criterion is that cc2 and/or cc3 is less than 5 hours.

9. The method according to any of the preceding items, wherein a quality criterion is determination of the time for cleavage to a 2 blastomere embryo, a 3 blastomere embryo, a 4 blastomere embryo, a 5 blastomere embryo, a 6 blastomere embryo, a 7 blastomere embryo, and/or an 8 blastomere embryo.

10. The method according to any of the preceding items, wherein the quality criterion is selected from the group of normalized morphological embryo parameters.

11. The method according to any of the preceding items, wherein the quality criterion is selected from the group of normalized morphological embryo parameters relating to the phase of from 2 to 8 blastomere embryo.

12. The method according to any of the preceding items, wherein a quality criterion is a normalized morphological embryo parameter based on two, three, four, five or more parameters selected from the group of t2, t3, t4, t5, t6, t7 and t8.

13. The method according to any of the preceding items, wherein a quality criterion is a normalized morphological embryo parameter based on four parameters selected from the group of t2, t3, t4, t5, t6, t7 and t8.

14. The method according to any of the preceding items, wherein a quality criterion is based on the ratio of two time intervals, each of said time intervals determined as the duration of a time period between two morphological events in the embryo development.

15. The method according to item 14, wherein said quality criterion is a normalized morphological embryo parameter.

16. The method according to any of the preceding items 14 to 15, wherein said morphological events are selected from the group of fertilization, initiation of a blastomere cleavage and completion of a blastomere cleavage.

17. The method according to any of the preceding items 10 to 16, wherein the normalized morphological embryo parameter is selected from the group of

$$cc2/cc2\_3 = (t3-t2)/(t5-t2),$$

$$cc3/cc2\_3 = (t5-t3)/(t5-t2),$$

$$cc3/t5 = 1 - t3/t5,$$

$$s2/cc2 = (t4-t3)/(t3-t2),$$

$$s3/cc3 = (t8-t5)/(t5-t3),$$

and

$$cc2/cc3 = (t3-t2)/(t5-t3).$$

18. The method according to any of the preceding items, wherein a quality criterion is determination of cc2/cc2_3 = (t3-t2)/(t5-t2).

19. The method according to item 18, wherein said quality criterion is an indicator of high embryo quality if cc2/cc2_3 = (t3-t2)/(t5-t2) is between 0.38 and 0.5, or between 0.39 and 0.49, or between 0.4 and 0.48 or between 0.41 and 0.47.

20. The method according to any of the preceding items, wherein the quality criterion is determination of t3/t5.

21. The method according to item 20, wherein said quality criterion is an indicator of high embryo quality if t3/t5 is greater than 0.6, or greater than 0.62, or greater than 0.64, or greater than 0.66, or greater than 0.68, or greater than 0.7, or greater than 0.72, or greater than 0.74.

22. The method according to any of the preceding items, wherein a quality criterion is determination of s2/cc2 = (t4-t3)/(t3-t2).

23. The method according to item 22, wherein said quality criterion is an indicator of high embryo quality if s2/cc2 = (t4-t3)/(t3-t2) is less than 0.03, or less than 0.029, or less than 0.028, or less than 0.027, or less than 0.026, or less than 0.025, or less than 0.024, or less than 0.023, or less than 0.022, or less than 0.021, or less than 0.02.

24. The method according to any of the preceding items, wherein a quality criterion is determination of s3/cc3 = (t8-t5)/(t5-t3).

25. The method according to item 22, wherein said quality criterion is an indicator of high embryo quality if s3/cc3 = (t8-t5)/(t5-t3) is less than 0.25, or less than 0.23, or less than 0.21, or less than 0.2, or less than 0.19, or less than 0.18, or less than 0.17, or less than 0.16, or less than 0.15.

26. The method according to any of the preceding items, wherein a quality criterion is determination of cc2/cc3 = (t3-t2)/(t5-t3).

27. The method according to item 26, wherein said quality criterion is an indicator of high embryo quality if cc2/cc3 = (t3-t2)/(t5-t3) is between 0.7 and 0.9, or between 0.71 and 0.89, or between 0.72 and 0.88.

28. The method according to any of the preceding items, wherein a quality criterion is determination of the extent of irregularity of the timing of cell divisions when the embryo develops from 4 to 8 blastomeres.

29. The method according to any of the preceding items, wherein a quality criterion is determination of the maximum cleavage time for each blastomere when the embryo develops from 4 to 8 blastomeres.

30. The method according to item 29, wherein said quality criterion is an indicator of high embryo quality if said maximum cleavage time is less than 1.5 hours.

31. The method according to item 29, wherein said quality criterion is an indicator of high embryo quality if said maximum cleavage time is less than 2.5 hours, or less than 2.3 hours, or less than 2.1 hours, or less than 2 hours, or less than 1.9 hours, or less than 1.8 hours, or less than 1.7 hours, or less than 1.65 hours, or less than 1.6 hours, or less than 1.55 hours, or less than 1.5 hours, or less than 1.45 hours, or less than 1.4 hours, or less than 1.35 hours, or less than 1.3 hours, or less than 1.25 hours, or less than 1.2 hours, or less than 1.15 hours, or less than 1.1 hours, or less than 1 hour.

32. The method according to any of the preceding items, wherein a quality criterion is determination of the ratio between the maximum cleavage time for each blastomere when the embryo develops from 4 to 8 blastomeres and the duration of the total time period from 4 to 8 blastomeres; max(s3a,s3b,s3c)/s3.

33. The method according to item 32, wherein said quality criterion is a normalized morphological embryo parameter.

34. The method according to any of the preceding items 32 to 33, wherein said quality criterion is an indicator of high embryo quality if said ratio is less than 0.5.

35. The method according to any of the preceding items 32 to 33, wherein said quality criterion is an indicator of high embryo quality if said ratio is less than 0.8, or less than 0.75, or less than 0.7, or less than 0.65, or less than 0.6, or less than 0.58, or less than 0.56, or less than 0.54, or less than 0.52, or less than 0.5, or less than 0.48, or less than 0.46, or less than 0.44, or less than 0.42, or less than 0.4.

36. The method according to any of the preceding items, wherein a quality criterion is determination of the time for cleavage to a 5 blastomere embryo.

37. The method according to item 36, wherein said quality criterion is an indicator of high embryo quality if t5 is less than 58 hours, or less than 57 hours or less than 56.5 hours, or less than 56.3 hours, or less than 56.2 hours, or less than 56.1 hours, or less than 56 hours, or less than 55.9 hours, or less than 55.8 hours, or less than 55.7 hours, or less than 55.6 hours, or less than 55.5 hours, or less than 55 hours, or less than 54.5 hours

38. The method according to any of the preceding items 36 to 37, wherein said quality criterion is an indicator of high embryo quality if t5 is greater than 46 hours, or greater than 47 hours, or greater than 47 hours, or greater than 48 hours, or greater than 48.5 hours, or greater than 48.7 hours, or greater than 48.9 hours, or greater than 49 hours, or greater than 49.1 hours, or greater than 49.2 hours, or greater than 49.3 hours, or greater than 49.4 hours, or greater than 49.5 hours, or greater than 49.6 hours, or greater than 49.7 hours,
or greater than 49.8 hours, or greater than 49.9 hours, or greater than 50 hours, or greater than 51 hours, or greater than 52 hours, or greater than 53 hours.

39. The method according to item 36, wherein said quality criterion is an indicator of high embryo quality ratio if t5 is between 48.7 and 55.6 hours.

40. The method according to any of the preceding items, wherein a quality criterion is determination of the time for cleavage to an 8 blastomere embryo, t8.

41. The method according to item 36, wherein said quality criterion is an indicator of high embryo quality if t8 is less than 60 hours, or less than 59 hours or less than 58 hours, or less than 57.8 hours, or less than 57.6 hours, or less than 57.4 hours, or less than 57.2 hours, or less than 57 hours, or less than 56.8 hours, or less than 56.6 hours, or less than 56.4 hours, or less than 56.2 hours, or less than 56 hours, or less than 55 hours.

42. The method according to any of the preceding items, wherein a quality criterion is determination of the second cell cycle length cc2.

43. The method according to item 42, wherein said quality criterion is an indicator of high embryo quality if cc2 = t3-t2 is less than 14 hours, or less than 13.5 hours, or less than 13 hours, or less than 12.9 hours, or less than 12.8 hours, or less than 12.7 hours, or less than 12.6 hours, or less than 12.5 hours, or less than 12.4 hours, or less than 12.3 hours, or less than 12.1 hours, or less than 12 hours, or less than 11.9 hours, or less than 11.9 hours, or less than 11.8 hours, or less than 11.7 hours, or less than 11.6 hours, or less than 11.5 hours, or less than 11.4 hours, or less than 11.3 hours, or less than 11.2 hours, or less than 11.1 hours, or less than 11 hours, or less than 10.9 hours, or less than 10.8 hours, or less than 10.7 hours, or less than 10.6 hours, or less than 10.5 hours, or less than 10 hours.

44. The method according to any of the preceding items, wherein a quality criterion is determination of cc2b = t4-t2.

45. The method according to item 44, wherein said quality criterion is an indicator of high embryo quality if cc2b = t4-t2 is less than 14 hours, or less than 13.9 hours, or less than 13.8 hours, or less than 13.7 hours, or less than 13.6 hours, or less than 13.5 hours, or less than 13.4 hours, or less than 13.3 hours, or less than 13.2 hours, or less than 13.1 hours, or less than 13 hours, or less than 12.9 hours, or less than 12.8 hours, or less than 12.7 hours, or less than 12.6 hours, or less than 12.5 hours, or less than 12.4 hours, or less than 12.3 hours, or less than 12.1 hours, or less than 12 hours, or less than 11.9 hours, or less than 11.9 hours, or less than 11.8 hours, or less than 11.7 hours, or less than 11.6 hours, or less than 11.5 hours, or less than 11.4 hours, or less than 11.3 hours, or less than 11.2 hours, or less than 11.1 hours, or less than 11 hours, or less than 10.9 hours, or less than 10.8 hours, or less than 10.7 hours, or less than 10.6 hours, or less than 10.5 hours, or less than 10 hours.

46. The method according to any of the preceding items, wherein a quality criterion is determination of the third cell cycle length cc3.

47. The method according to item 46, wherein said quality criterion is an indicator of high embryo quality if cc3 = t5-t3 is less than 19 hours, or less than 18.5 hours, or less than 18 hours, or less than 17.9 hours, or less than 17.8 hours, or less than 17.7 hours, or less than 17.6 hours, or less than 17.5 hours, or less than 17.4 hours, or less than 17.3 hours, or less than 17.2 hours, or less than 17.1 hours, or less than 17 hours, or less than 16.9 hours, or less than 16.8 hours, or less than 16.7 hours, or less than 16.6 hours, or less than 16.5 hours, or less than 16.4 hours, or less than 16.3 hours, or less than 16.2 hours, or less than 16.1 hours, or less than 16 hours, or less than 15.8 hours, or less than 15.6 hours, or less than 15.5 hours, or less than 15.4 hours, or less than 15.3 hours, or less than 15.1 hours, or less than 15 hours, or less than 14.9 hours, or less than 14.9 hours, or less than 14.8 hours, or less than 14.7 hours, or less than 14.6 hours, or less than 14.5 hours, or less than 14.4 hours, or less than 14.3 hours, or less than 14.2 hours, or less than 14.1 hours, or less than 14 hours, or less than 13 hours.

48. The method according to any of items 46 to 47, wherein said quality criterion is an indicator of high embryo quality if cc3 = t5-t3 is greater than 11 hours, or greater than 11.5 hours, or greater than 12 hours, or greater than 12.2 hours, or greater than 12.4 hours, or greater than 12.5 hours, or greater than 12.6 hours, or greater than 12.7 hours, or greater than 12.8 hours, or greater than 12.9 hours, or greater than 13 hours, or greater than 13.1 hours, or greater than 13.2 hours, or greater than 13.3 hours, or greater than 13.5 hours, or greater than 14 hours.

49. The method according to any of the preceding items, wherein a quality criterion is determination of cc2_3 = t5-t2.

50. The method according to item 49, wherein said quality criterion is an indicator of high embryo quality if cc2_3 = t5-t2 is less than 32 hours, or less than 31 hours, or less than 30 hours, or less than 29.8 hours, or less than 29.6 hours, or less than 29.5 hours, or less than 29.4 hours, or less than 29.3 hours, or less than 29.2 hours, or less than 29.1 hours, or less than 29 hours, or less than 28.9 hours, or less than 28.8 hours, or less than 28.7 hours, or less than 28.6 hours, or less than 28.5 hours, or less than 28.4 hours, or less than 28.2 hours, or less than 28 hours, or less than 27.5 hours, or less than 27 hours, or less than 26 hours.

51. The method according to any of the preceding items, wherein a quality criterion is determination of the synchrony in division from a 2 blastomere embryo to a 4 blastomere embryo s2 = t4-t3.

52. The method according to item 51, wherein said quality criterion is an indicator of high embryo quality if s2 = t4-t3 is less than 3 hours, or less than 2.8 hours, or less than 2.6 hours, or less than 2.4 hours, or less than 2.3 hours, or less than 2.2 hours, or less than 2.1 hours, or less than 2 hours, or less than 1.8 hours, or less than 1.6 hours, or less than 1.4 hours, or less than 1.2 hours, or less than 1 hour, or less than 0.9 hours, or less than 0.8 hours, or less than 0.7 hours, or less than 0.6 hours, or less than 0.5 hours, or less than 0.45 hours, or less than 0.4 hours, or less than 0.39 hours, or less than 0.38 hours, or less than 0.37 hours, or less than 0.36 hours, or less than 0.35 hours, or less than 0.34 hours, or less than 0.33 hours, or less than 0.32 hours, or less than 0.31 hours, or less than 0.3 hours, or less than 0.29 hours, or less than 0.28 hours, or less than 0.27 hours, or less than 0.26 hours, or less than 0.25 hours, or less than 0.24 hours, or less than 0.22 hours, or less than 0.2 hours.

53. The method according to any of the preceding items, wherein a quality criterion is determination of the synchrony in division from a 4 blastomere embryo to a 8 blastomere embryo s3 = t8-t5.

54. The method according to item 53, wherein said quality criterion is an indicator of high embryo quality if s3 = t8-t3 is less than 5 hours, or less than 4.5 hours, or less than 4.3 hours, or less than 4.2 hours, or less than 4.1 hours, or less than 4 hours, or less than 3.9 hours, or less than 3.8 hours, or less than 3.7 hours, or less than 3.6 hours, or less than 3.5 hours, or less than 3.4 hours, or less than 3.3 hours, or less than 3.2 hours, or less than 3.1 hours, or less than 3 hours, or less than 2.9 hours, or less than 2.8 hours, or less than 2.7 hours, or less than 2.6 hours, or less than 2.55 hours, or less than 2.53 hours, or less than 2.51 hours, or less than 2.5 hours, or less than 2.4 hours, or less than 2.3 hours, or less than 2.2 hours, or less than 2.1 hours, or less than 2 hours, or less than 1.8 hours, or less than 1.6 hours, or less than 1.4 hours, or less than 1.2 hours, or less than 1 hour.

55. The method according to any of the preceding items, wherein the quality criterion is combined with determination of second cell cycle length.

56. The method according to any of the preceding items, wherein the quality criterion is combined with determination of synchrony in cleavage from a 2 blastomere embryo to a 4 blastomere embryo.

57. The method according to any of the preceding items, wherein the quality criterion is a combination of determination of time for cleavage to a 5 blastomere embryo and determination of the second cell cycle length.

58. The method according to item 9, where the quality criterion is further combined with determination of synchrony in cleavage from 2 blastomere embryo to 4 blastomere embryo.

59. The method according to any of the preceding items, wherein the determination of embryo quality further includes i) determining the extent and/or spatial distribution of cellular or organelle movement during the cell cleavage period; and/or ii) determining the extent and/or spatial distribution of cellular or organelle movement during the inter-cleavage period thereby obtaining an embryo quality measure.

60. The method according to any of the preceding items, wherein the embryo is monitored for a time period comprising at least three cell cycles, such as at least four cell cycles.

61. The method according to any of the preceding items, wherein the length of each cleavage period is determined.

62. The method according to any of the preceding items, wherein the length of each inter-cleavage period is determined.

63. The method according to any of the preceding items, wherein the period of cellular movement in at least two inter-cleavage periods is determined.

64. The method according to any of the preceding items, wherein the extent of cellular movement is determined in at least two inter-cleavage periods.

65. The method according to any of the preceding items, wherein the quality measure includes at least one exclusion criterion.

66. The method according to any of preceding items, wherein the exclusion criterion includes information of blastomere evenness at t2, information of multi nuclearity at the two-blastomere stage and/or at the four-blastomere stage, and/or information of cleavage from one blastomere directly to three blastomeres.

67. The method according to any of the preceding items, wherein an exclusion criterion is that cc2 and/or cc3 is less than 10 hours, or less than 9.5 hours, or less than 9 hours, or less than 8.5 hours, or less than 8 hours, or less than 7.5 hours, or less than 7 hours, or less than 6.5 hours, or less than 6 hours, or less than 5.5 hours, or less than 5 hours, or less than 4.5 hours, or less than 4 hours, or less than 3.5 hours, or less than 3 hours, or less than 2.5 hours, or less than 2 hours, or less than 1.5 hours, or less than 1 hour.

68. The method according to any of the preceding items, wherein an exclusion criterion is that t2 is greater than 28 hours, or greater than 28.5 hours, or greater than 29 hours, or greater than 29.5 hours, or greater than 30 hours, or greater than 30.5 hours, or greater than 31 hours, or greater than 31.25 hours, or greater than 31.5 hours, or greater than 31.75 hours, or greater than 32 hours, or greater than 32.5 hours, or greater than 33 hours, or greater than 33.5 hours, or greater than 34 hours.

69. The method according to any of the preceding items, wherein an exclusion criterion is that cc2b is greater than 11 hours, or greater than 11.5 hours, or greater than 12 hours, or greater than 12.5 hours, or greater than 12.75 hours, or greater than 13 hours, or greater than 13.1 hours, or greater than 13.25 hours, or greater than 13.5 hours, or greater than 14 hours, or greater than 14.5 hours, or greater than 15 hours.

70. The method according to any of the preceding items, wherein an exclusion criterion is that cc3 is greater than 15 hours, or greater than 15.5 hours, or greater than 16 hours, or greater than 16.5 hours, or greater than 17 hours, or greater than 17.25 hours, or greater than 17.5 hours, or greater than 17.6 hours, or greater than 17.75 hours, or greater than 18 hours, or greater than 18.5 hours, or greater than 19 hours, or greater than 19.5 hours.

71. The method according to any of the preceding items, wherein an exclusion criterion is that s2 is greater than 1 hour, or greater than 1.1 hours, or greater than 1.2 hours, or greater than 1.3 hours, or greater than 1.4 hours, or greater than 1.5 hours, or greater than 1.6 hours, or greater than 1.7 hours, or greater than 1.8 hours, or greater than 1.9 hours, or greater than 2 hours, or greater than 2.1 hours, or greater than 2.2 hours, or greater than 2.3 hours, or greater than 2.4 hours, or greater than 2.5 hours, or greater than 2.6 hours, or greater than 2.7 hours, or greater than 2.8 hours, or greater than 2.9 hours, or greater than 3 hours.

72. The method according to any of the preceding items, wherein an exclusion criterion is that s3 is greater than 2 hours, or greater than 2.2 hours, or greater than 2.4 hours, or greater than 2.6 hours, or greater than 2.8 hours, or greater than 3 hours, than 3.1 hours, or greater than 3.2 hours, or greater than 3.3 hours, or greater than 3.4 hours, or greater than 3.5 hours, or greater than 3.6 hours, or greater than 3.7 hours, or greater than 3.8 hours, or greater than 3.9 hours, or greater than 4 hours, than 4.1 hours, or greater than 4.2 hours, or greater than 4.3 hours, or greater than 4.4 hours, or greater than 4.5 hours, or greater than 4.6 hours, or greater than 4.7 hours, or greater than 4.8 hours, or greater than 4.9 hours, or greater than 5 hours, or greater than 5.25 hours, or greater than 5.5 hours, or greater than 6 hours.

73. The method according to any of the preceding items, wherein the embryo is monitored in an incubator.

74. The method according to any of the preceding items, wherein the embryo is monitored through image acquisition, such as image acquisition at least once per hour, preferably image acquisition at least once per half hour.

75. A method for selecting an embryo suitable for transplantation, said method comprising monitoring the embryo as defined in any of the items 1-74 obtaining an embryo quality measure, and selecting the embryo having the highest embryo quality measure.

76. A system for determining embryo quality comprising means for monitoring the embryo for a time period, said system further having means for determining a quality criteria for said embryo, and having means for determining the embryo quality based on said quality criteria.

77. The system according to item 76, comprising means for determining one or more of the features as defined in any of the items 1-74.

Further aspects of the invention are set out in the following numbered clauses.

1. A method for determining embryo quality comprising monitoring the embryo for a time period, and determining one or more quality criteria for said embryo, wherein said one or more quality criteria is based on the extent of irregularity of the timing of cell divisions when the embryo develops from four to eight blastomeres, and/or wherein said one or more quality criteria is based on determining the time of cleavage to a five blastomere embryo (t5) and wherein t5 is between 48.7 hours and 55.6 hours, and/or wherein said one or more quality criteria is based on the ratio of two time intervals, each of said two time intervals determined as the duration of a time period between two morphological events in the embryo development from fertilization to eight blastomeres, and based on said one or more quality criteria determining the embryo quality.

2. The method according to clause 1, wherein the embryo quality is determined from a plurality of said quality criteria, such as by combining a plurality of said quality criteria.

3. The method according to any of the preceding clauses, wherein the embryo quality is a quality relating to implantation success.

4. The method according to any of the preceding clauses, wherein said one or more quality criteria are combined with one or more exclusion criteria for deselecting and/or excluding embryos with a low probability of implantation success.

5. The method according to clause 4, wherein an exclusion criterion is that cc2 and/or cc3 is less than 5 hours.

6. The method according to any of the preceding clauses, wherein each of said two time intervals is based on one parameter, or subtraction of two parameters, selected from the group of t2, t3, t4, t5, t6, t7 and t8.

7. The method according to any of the preceding clauses, wherein said morphological events are selected from the group of fertilization, initiation of a blastomere cleavage and completion of a blastomere cleavage.

8. The method according to any of the preceding clauses wherein said ratio of two time intervals is selected from the group of:

$$cc2/cc2\_3 = (t3-t2)/(t5-t2),$$

$$cc3/cc2\_3 = (t5-t3)/(t5-t2),$$

$$cc3/t5 = 1 - t3/t5,$$

$$s2/cc2 = (t4-t3)/(t3-t2),$$

$$s3/cc3 = (t8-t5)/(t5-t3),$$

and

$$cc2/cc3 = (t3-t2)/(t5-t3).$$

9. The method according to any of the preceding clauses, wherein a quality criterion is determination of cc2/cc2_3 = (t3-t2)/(t5-t2) and wherein said quality criterion is an indicator of high embryo quality if cc2/cc2_3 between 0.41 and 0.47.

10. The method according to any of the preceding clauses, wherein a quality criterion is determination of cc3/t5 = 1-t3/t5 and wherein said quality criterion is an indicator of high embryo quality if cc3/t5 is between 0.26 and 0.28.

11. The method according to any of the preceding clauses, wherein a quality criterion is determination of s2/cc2 = (t4-t3)/(t3-t2) and wherein said quality criterion is an indicator of high embryo quality if s2/cc2 = (t4-t3)/(t3-t2) is less than 0.025.

12. The method according to any of the preceding clauses, wherein a quality criterion is determination of s3/cc3 = (t8-t5)/(t5-t3) and wherein said quality criterion is an indicator of high embryo quality if s3/cc3 is less than 0.18.

13. The method according to any of the preceding clauses, wherein a quality criterion is determination of cc2/cc3 = (t3-t2)/(t5-t3) and wherein said quality criterion is an indicator of high embryo quality if cc2/cc3 is between 0.72 and 0.88.

14. The method according to any of the preceding clauses, wherein said extent of irregularity of the timing of cell divisions when the embryo develops from four to eight blastomeres is determined by calculating the maximum cleavage time for each blastomere when the embryo develops from 4 to 5 to 6 to 7 and to 8 blastomeres.

15. The method according to clause 14, wherein said quality criterion is an indicator of high embryo quality if said maximum cleavage time is less than 1.5 hours.

16. The method according to any of the preceding clauses, wherein said extent of irregularity of the timing of cell divisions when the embryo develops from four to eight blastomeres is determined by calculating the ratio between the maximum cleavage time for each blastomere when the embryo develops from 4 to 5 to 6 to 7 and to 8 blastomeres and the duration of the total time period from 4 to 8 blastomeres; max(s3a,s3b,s3c)/s3.

17. The method according to clause 16, wherein said quality criterion is an indicator of high embryo quality if said ratio is less than 0.5.

18. The method according to any of the preceding clauses, wherein a quality criterion is determination of the time for cleavage to an 8 blastomere embryo, t8 and wherein said quality criterion is an indicator of high embryo quality if t8 is less than 57.2 hours.

19. The method according to any of the preceding clauses, wherein a quality criterion is determination of the second cell cycle length cc2 = t3-t2 and wherein said quality criterion is an indicator of high embryo quality if cc2 is less than 12.7 hours.

20. The method according to any of the preceding clauses, wherein a quality criterion is determination of cc2b = t4-t2 and wherein said quality criterion is an indicator of high embryo quality if cc2b is less than 12.7 hours.

21. The method according to any of the preceding clauses, wherein a quality criterion is determination of the third cell cycle length cc3 = t5-t3 and wherein said quality criterion is an indicator of high embryo quality if cc3 is between 12.9 and 16.3 hours.

22. The method according to any of the preceding clauses, wherein a quality criterion is determination of cc2_3 = t5-t2 t3 and wherein said quality criterion is an indicator of high embryo quality if cc2_3 is between 24 and 28.7 hours.

23. The method according to any of the preceding clauses, wherein a quality criterion is determination of the synchrony in division from a 2 blastomere embryo to a 4 blastomere embryo s2 = t4-t3 and wherein said quality criterion is an indicator of high embryo quality if s2 is less than 1.33 hours or less than 0.33 hours.

24. The method according to any of the preceding clauses, wherein a quality criterion is determination of the synchrony in division from a 4 blastomere embryo to a 8 blastomere embryo s3 = t8-t5 and wherein said quality criterion is an indicator of high embryo quality if s3 is less than 2.7 hours.

25. The method according to any of the preceding clauses, wherein an exclusion criterion includes information of blastomere evenness at t2, information of multi nuclearity at the two blastomere stage and/or at the four-blastomere stage, and/or information of cleavage from one blastomere directly to three blastomeres.

26. The method according to any of the preceding clauses, wherein an exclusion criterion is that t2 is greater than 31.8 hours.

27. The method according to any of the preceding clauses, wherein an exclusion criterion is that t5 is less than 49 hours.

28. The method according to any of the preceding clauses, wherein an exclusion criterion is that cc2b is greater than 13.1 hours.

29. The method according to any of the preceding clauses, wherein an exclusion criterion is that cc3 is greater than 17.6 hours.

30. The method according to any of the preceding clauses, wherein an exclusion criterion is that s2 is greater than 2.1 hours.

31. The method according to any of the preceding clauses, wherein an exclusion criterion is that s3 is greater than 13.8 hours.

32. The method according to any of the preceding clauses, wherein the embryo is monitored in an incubator.

33. The method according to any of the preceding clauses, wherein the embryo is monitored through image acquisition, such as image acquisition at least once per hour, preferably image acquisition at least once per half hour.

34. A method for selecting an embryo suitable for transplantation, said method comprising monitoring the embryo as defined in any of the clauses 1-33 obtaining an embryo quality measure, and selecting the embryo having the highest embryo quality measure.

35. A system for determining embryo quality comprising means for monitoring the embryo for a time period, said system further having means for determining a quality criteria for said embryo, and having means for determining the embryo quality based on said quality criteria.

36. The system according to clause 35, comprising means for determining one or more of the features as defined in any of the clauses 1-33.

**Claims**

1. A method for determining human embryo quality comprising monitoring the human embryo for a time period after in vitro fertilization, and determining one or more quality criteria for said human embryo, wherein said one or more quality criteria is selected from the group of

$$cc2/cc3 = (t3-t2)/(t5-t3),$$

$$cc2/cc2\_3 = (t3-t2)/(t5-t2),$$

and

$$cc3/cc2\_3 = (t5-t3)/(t5-t2),$$

and based on said one or more quality criteria determining the human embryo quality,

wherein t2 is a time of cleavage to a 2 blastomere embryo; t3 is a time of cleavage to a 3 blastomere embryo; t5 is a time of cleavage to a 5 blastomere embryo; cc2 is t3-t2; cc3 is t5-t3; and cc2_3 is t5-t2, wherein said one or more quality criteria is an indicator of high embryo quality if
cc2/cc3 is between 0.7 and 0.9 and/or
cc2/cc2_3 is between 0.38 and 0.5 and/or
cc3/cc2_3 is between 0.5 and 0.62.

2. The method according to claim 1, wherein the human embryo quality is determined from a plurality of said quality criteria, such as by combining a plurality of said quality criteria.

3. The method according to any of the preceding claims, wherein the human embryo quality is a quality relating to implantation success.

4. The method according to any of the preceding claims, wherein said one or more quality criteria are combined with one or more exclusion criteria for deselecting and/or excluding human embryos with a low probability of implantation success.

5. The method according to claim 4, wherein an exclusion criterion is that cc2 and/or cc3 is less than 5 hours.

6. The method according to any of the preceding claims wherein a further quality criteria is selected from the group of:

$$cc3/t5 = 1 - t3/t5,$$

$$s2/cc2 = (t4-t3)/(t3-t2),$$

and

$$s3/cc3 = (t8-t5)/(t5-t3),$$

wherein t4 is a time of cleavage to a 4 blastomere embryo; t8 is a time of cleavage to an 8 blastomere embryo; S2 is t4-t3; and S3 is t8-t5.

7. The method according to any of the preceding claims,

wherein a further quality criterion is determination of cc3/t5 = 1-t3/t5 and wherein said quality criterion is an indicator of high embryo quality if cc3/t5 is between 0.26 and 0.28; and / or
wherein a further quality criterion is determination of s2/cc2 = (t4-t3)/(t3-t2) and wherein said quality criterion is an indicator of high embryo quality if s2/cc2 = (t4-t3)/(t3-t2) is less than 0.025; and / or wherein a further quality criterion is determination of s3/cc3 = (t8-t5)/(t5-t3) and wherein said quality criterion is an indicator of high embryo quality if s3/cc3 is less than 0.18; and / or
wherein a further quality criterion is determination of the time for cleavage to an 8 blastomere embryo, t8 and wherein said quality criterion is an indicator of high embryo quality if t8 is less than 57.2 hours; and / or
wherein a further quality criterion is determination of the second cell cycle length cc2 = t3-t2 and wherein said quality criterion is an indicator of high embryo quality if cc2 is less than 12.7 hours; and / or
wherein a further quality criterion is determination of cc2b = t4-t2 and wherein said quality criterion is an indicator of high embryo quality if cc2b is less than 12.7 hours; and / or
wherein a further quality criterion is determination of the third cell cycle length cc3 = t5-t3 and wherein said quality criterion is an indicator of high embryo quality if cc3 is between 12.9 and 16.3 hours; and / or
wherein a further quality criterion is determination of cc2_3 = t5- t3 and wherein said quality criterion is an indicator of high embryo quality if cc2_3 is between 24 and 28.7 hours; and / or wherein a further quality criterion is determination of the synchrony in division from a 2 blastomere embryo to a 4 blastomere embryo s2 = t4-t3 and wherein said quality criterion is an indicator of high embryo quality if s2 is less than 1.33 hours or less than 0.33 hours; and / or wherein a further quality criterion is determination of the synchrony in division from a 4 blastomere embryo to a 8 blastomere embryo s3 = t8-t5 and wherein said quality criterion is an indicator of high embryo quality if s3 is less than 2.7 hours,
wherein t4 is a time of cleavage to a 4 blastomere embryo; t8 is a time of cleavage to an 8 blastomere embryo; cc2b is t4-t2; S2 is t4-t3; and S3 is t8-t5.

8. The method according to any of the preceding claims, wherein an exclusion criterion includes information of blastomere evenness at t2, information of multi nuclearity at the two blastomere stage and/or at the four-blastomere stage, and/or information of cleavage from one blastomere directly to three blastomeres.

9. The method according to any of the preceding claims,

wherein an exclusion criterion is that t2 is greater than 31.8 hours; and / or
wherein an exclusion criterion is that t5 is less than 49 hours; and / or
wherein an exclusion criterion is that cc2b is greater than 13.1 hours; and / or
wherein an exclusion criterion is that cc3 is greater than 17.6 hours; and / or
wherein an exclusion criterion is that s2 is greater than 2.1 hours; and / or
wherein an exclusion criterion is that s3 is greater than 13.8 hours,
wherein cc2b is t4-t2; t4 is a time of cleavage to a 4 blastomere embryo; S2 is t4-t3; and S3 is t8-t5; and t8 is a time of cleavage to an 8 blastomere embryo.

10. The method according to any of the preceding claims, wherein the human embryo is monitored in an incubator.

**11.** The method according to any of the preceding claims, wherein the human embryo is monitored through image acquisition, such as image acquisition at least once per hour, preferably image acquisition at least once per half hour.

**Patentansprüche**

**1.** Verfahren zur Bestimmung der Qualität eines menschlichen Embryos, umfassend das Überwachen des menschlichen Embryos für einen Zeitraum nach der In-vitro-Fertilisation und das Bestimmen eines Qualitätskriteriums oder mehrerer Qualitätskriterien für den menschlichen Embryo, wobei das eine Qualitätskriterium oder mehrere Qualitätskriterien ausgewählt ist/sind aus der Gruppe von

$$cc2/cc3 = (t3-t2)/(t5-t3),$$

$$cc2/cc2\_3 = (t3-t2)/(t5-t2)$$

und

$$cc3/cc2\_3 = (t5-t3)/(t5-t2)$$

und auf der Grundlage von diesem einen Qualitätskriterium oder mehreren Qualitätskriterien, das/die die Qualität des menschlichen Embryos bestimmt/bestimmen,

wobei t2 die Teilungsdauer zu einem 2-Blastomeren-Embryo ist; t3 die Teilungsdauer zu einem 3-Blastomeren-Embryo ist; t5 die Teilungsdauer zu einem 5-Blastomeren-Embryo ist; cc2 gleich t3-t2 ist; cc3 gleich t5-t3 ist; und cc2_3 gleich t5-t2 ist, wobei das eine Qualitätskriterium oder mehrere Qualitätskriterien ein Indikator für eine hohe Embryoqualität ist/sind, wenn
cc2/cc3 zwischen 0,7 und 0,9 liegt und/oder
cc2/cc2_3 zwischen 0,38 und 0,5 liegt und/oder
cc3/cc2_3 zwischen 0,5 und 0,62 liegt.

**2.** Verfahren nach Anspruch 1, wobei die Qualität des menschlichen Embryos aus einer Vielzahl von Qualitätskriterien bestimmt wird, wie durch Kombinieren einer Vielzahl von Qualitätskriterien.

**3.** Verfahren nach einem der vorhergehenden Ansprüche, wobei die Qualität des menschlichen Embryos eine Qualität im Zusammenhang mit dem Implantationserfolg ist.

**4.** Verfahren nach einem der vorhergehenden Ansprüche, wobei das eine Qualitätskriterium oder mehrere Qualitätskriterien mit einem oder mehreren Ausschlusskriterien zum Deselektieren und/oder Ausschließen von menschlichen Embryonen mit einer geringen Wahrscheinlichkeit des Implantationserfolgs kombiniert wird/werden.

**5.** Verfahren nach Anspruch 4, wobei ein Ausschlusskriterium ist, dass cc2 und/oder cc3 weniger als 5 Stunden beträgt/betragen.

**6.** Verfahren nach einem der vorhergehenden Ansprüche, wobei ein weiteres Qualitätskriterium ausgewählt ist aus der Gruppe:

$$cc3/t5 = 1 - t3/t5,$$

$$s2/cc2 = (t4-t3)/(t3-t2)$$

und

$$s3/cc3 = (t8-t5)/(t5-t3),$$

wobei t4 die Teilungsdauer zu einem 4-Blastomeren-Embryo ist; t8 die Teilungsdauer zu einem 8-Blastomeren-Embryo ist; S2 gleich t4-t3 ist; und S3 gleich t8-t5 ist.

7. Verfahren nach einem der vorhergehenden Ansprüche,
wobei ein weiteres Qualitätskriterium die Bestimmung von cc3/t5 = 1-t3/t5 ist und wobei das Qualitätskriterium ein Indikator für hohe Embryoqualität ist, wenn cc3/t5 zwischen 0,26 und 0,28 liegt; und/oder
wobei ein weiteres Qualitätskriterium die Bestimmung von s2/cc2 = (t4-t3)/(t3-t2) ist und wobei das Qualitätskriterium ein Indikator für eine hohe Embryoqualität ist, wenn s2/cc2 = (t4-t3)/(t3-t2) kleiner als 0,025 ist; und/oder
wobei ein weiteres Qualitätskriterium die Bestimmung von s3/cc3 = (t8-t5)/(t5-t3) ist und wobei das Qualitätskriterium ein Indikator für hohe Embryoqualität ist, wenn s3/cc3 kleiner als 0,18 ist; und/oder
wobei ein weiteres Qualitätskriterium die Bestimmung der Teilungsdauer zu einem 8-Blastomeren-Embryo t8 ist und wobei das Qualitätskriterium ein Indikator für eine hohe Embryoqualität ist, wenn t8 weniger als 57,2 Stunden beträgt; und/oder
wobei ein weiteres Qualitätskriterium die Bestimmung der zweiten Zellzykluslänge cc2 = t3-t2 ist und wobei das Qualitätskriterium ein Indikator für hohe Embryoqualität ist, wenn cc2 weniger als 12,7 Stunden beträgt; und/oder
wobei ein weiteres Qualitätskriterium die Bestimmung von cc2b = t4-t2 ist und wobei das Qualitätskriterium ein Indikator für eine hohe Embryoqualität ist, wenn cc2b weniger als 12,7 Stunden beträgt; und/oder
wobei ein weiteres Qualitätskriterium die Bestimmung der dritten Zellzykluslänge cc3 = t5-t3 ist und wobei das Qualitätskriterium ein Indikator für hohe Embryoqualität ist, wenn cc3 zwischen 12,9 und 16,3 Stunden liegt; und/oder
wobei ein weiteres Qualitätskriterium die Bestimmung von cc2_3 = t5-t3 ist und wobei das Qualitätskriterium ein Indikator für eine hohe Embryoqualität ist, wenn cc2_3 zwischen 24 und 28,7 Stunden liegt; und/oder
wobei ein weiteres Qualitätskriterium die Bestimmung der Teilungssynchronie von einem 2-Blastomeren-Embryo zu einem 4-Blastomeren-Embryo s2 = t4-t3 ist und wobei das Qualitätskriterium ein Indikator für hohe Embryoqualität ist, wenn s2 weniger als 1,33 Stunden oder weniger als 0,33 Stunden beträgt; und/oder
wobei ein weiteres Qualitätskriterium die Bestimmung der Teilungssynchronie von einem 4-Blastomeren-Embryo zu einem 8-Blastomeren-Embryo s3 = t8-t5 ist und wobei das Qualitätskriterium ein Indikator für eine hohe Embryoqualität ist, wenn s3 weniger als 2,7 Stunden beträgt,
wobei t4 die Teilungsdauer zu einem 4-Blastomeren-Embryo ist; t8 die Teilungsdauer zu einem 8-Blastomeren-Embryo ist; cc2b gleich t4-t2 ist; S2 gleich t4-t3 ist; und S3 gleich t8-t5 ist.

8. Verfahren nach einem der vorhergehenden Ansprüche, wobei ein Ausschlusskriterium Informationen über die Blastomeren-Gleichmäßigkeit bei t2, Information über die Mehrkernigkeit im Zwei-Blastomeren-Stadium und/oder im Vier-Blastomeren-Stadium und/oder die Information über die Teilung von einer Blastomere direkt zu drei Blastomeren enthält.

9. Verfahren nach einem der vorhergehenden Ansprüche,
wobei ein Ausschlusskriterium ist, dass t2 mehr als 31,8 Stunden beträgt; und/oder
wobei ein Ausschlusskriterium ist, dass t5 weniger als 49 Stunden beträgt; und/oder
wobei ein Ausschlusskriterium ist, dass cc2b mehr als 13,1 Stunden beträgt; und/oder
wobei ein Ausschlusskriterium ist, dass cc3 mehr als 17,6 Stunden beträgt; und/oder
wobei ein Ausschlusskriterium ist, dass s2 mehr als 2,1 Stunden beträgt; und/oder
wobei ein Ausschlusskriterium ist, dass s3 mehr als 13,8 Stunden beträgt,
wobei cc2b gleich t4-t2 ist; t4 eine Teilungsdauer zu einem 4-Blastomeren-Embryo ist; S2 gleich t4-t3 ist; und S3 gleich t8-t5 ist; und t8 eine Teilungsdauer zu einem 8-Blastomeren-Embryo ist.

10. Verfahren nach einem der vorhergehenden Ansprüche, wobei der menschliche Embryo in einem Inkubator überwacht wird.

11. Verfahren nach einem der vorhergehenden Ansprüche, wobei der menschliche Embryo durch Bilderfassung, wie Bilderfassung mindestens einmal pro Stunde, vorzugsweise Bilderfassung mindestens einmal pro halbe Stunde, überwacht wird.

**Revendications**

1. Procédé de détermination de qualité d'embryon humain comprenant la surveillance de l'embryon humain pendant une période après fécondation *in vitro,* et la détermination d'un ou plusieurs critères de qualité pour ledit embryon humain, dans lequel lesdits un ou plusieurs critères de qualité sont choisis dans le groupe de

$$cc2/cc3 = (t3-t2)/(t5-t3),$$

$$cc2/cc2\_3 = (t3-t2)/(t5-t2),$$

et

$$cc3/cc2\_3 = (t5-t3)/(t5-t2),$$

et sur la base desdits un ou plusieurs critères de qualité, la détermination de la qualité d'embryon humain,

dans lequel t2 est un temps de clivage pour un embryon à 2 blastomères ; t3 est un temps de clivage pour un embryon à 3 blastomères ; t5 est un temps de clivage pour un embryon à 5 blastomères ; cc2 est t3-t2 ; cc3 est t5-t3 ; et cc2_3 est t5-t2, dans lequel lesdits un ou plusieurs critères de qualité sont un indicateur de haute qualité d'embryon si
cc2/cc3 est compris entre 0,7 et 0,9 et/ou
cc2/cc2_3 est compris entre 0,38 et 0,5 et/ou
cc3/cc2_3 est compris entre 0,5 et 0,62.

2. Procédé selon la revendication 1, dans lequel la qualité d'embryon humain est déterminée à partir d'une pluralité desdits critères de qualité, par exemple par combinaison d'une pluralité desdits critères de qualité.

3. Procédé selon l'une quelconque des revendications précédentes, dans lequel la qualité d'embryon humain est une qualité liée au succès d'implantation.

4. Procédé selon l'une quelconque des revendications précédentes, dans lequel lesdits un ou plusieurs critères de qualité sont combinés avec un ou plusieurs critères d'exclusion pour désélectionner et/ou exclure des embryons humains avec une faible probabilité de succès d'implantation.

5. Procédé selon la revendication 4, dans lequel un critère d'exclusion est que cc2 et/ou cc3 est inférieur à 5 heures.

6. Procédé selon l'une quelconque des revendications précédentes dans lequel un critère de qualité supplémentaire est choisi dans le groupe de :

$$cc3/t5 = 1 - t3/t5,$$

$$s2/cc2 = (t4-t3)/(t3-t2),$$

et

$$s3/cc3 = (t8-t5)/(t5-t3),$$

dans lequel t4 est un temps de clivage pour un embryon à 4 blastomères ; t8 est un temps de clivage pour un embryon à 8 blastomères ; S2 est t4-t3 ; et S3 est t8-t5.

7. Procédé selon l'une quelconque des revendications précédentes,

dans lequel un critère de qualité supplémentaire est la détermination de cc3/t5 = 1-t3/t5 et dans lequel ledit critère de qualité est un indicateur de haute qualité d'embryon si cc3/t5 est compris entre 0,26 et 0,28 ; et/ou

dans lequel un critère de qualité supplémentaire est la détermination de s2/cc2 = (t4-t3)/(t3-t2) et dans lequel ledit critère de qualité est un indicateur de haute qualité d'embryon si s2/cc2 = (t4-t3) / (t3-t2) est inférieur à 0,025 ; et/ou

dans lequel un critère de qualité supplémentaire est la détermination de s3/cc3 = (t8-t5)/(t5-t3) et dans lequel ledit critère de qualité est un indicateur de haute qualité d'embryon si s3/cc3 est inférieur à 0,18 ; et/ou

dans lequel un critère de qualité supplémentaire est la détermination du temps de clivage pour un embryon à 8 blastomères, t8 et dans lequel ledit critère de qualité est un indicateur de haute qualité d'embryon si t8 est inférieur à 57,2 heures ; et/ou

dans lequel un critère de qualité supplémentaire est la détermination de la durée du deuxième cycle cellulaire cc2 = t3-t2 et dans lequel ledit critère de qualité est un indicateur de haute qualité d'embryon si cc2 est inférieur à 12,7 heures ; et/ou

dans lequel un critère de qualité supplémentaire est la détermination de cc2b = t4-t2 et dans lequel ledit critère de qualité est un indicateur de haute qualité d'embryon si cc2b est inférieur à 12,7 heures ; et/ou

dans lequel un critère de qualité supplémentaire est la détermination de la durée du troisième cycle cellulaire cc3 = t5-t3 et dans lequel ledit critère de qualité est un indicateur de haute qualité d'embryon si cc3 est compris entre 12,9 et 16,3 heures ; et/ou

dans lequel un critère de qualité supplémentaire est la détermination de cc2_3 = t5-t3 et dans lequel ledit critère de qualité est un indicateur de haute qualité d'embryon si cc2_3 est compris entre 24 et 28,7 heures ; et/ou

dans lequel un critère de qualité supplémentaire est la détermination du synchronisme de division d'un embryon à 2 blastomères à un embryon à 4 blastomères s2 = t4-t3 et dans lequel ledit critère de qualité est un indicateur de haute qualité d'embryon si s2 est inférieur à 1,33 heure ou inférieur à 0,33 heure ; et/ou

dans lequel un critère de qualité supplémentaire est la détermination du synchronisme de division d'un embryon à 4 blastomères à un embryon à 8 blastomères s3 = t8-t5 et dans lequel ledit critère de qualité est un indicateur de haute qualité d'embryon si s3 est inférieur à 2,7 heures,

dans lequel t4 est un temps de clivage pour un embryon à 4 blastomères ; t8 est un temps de clivage pour un embryon à 8 blastomères ; cc2b est t4-t2 ; S2 est t4-t3 ; et S3 est t8-t5.

8. Procédé selon l'une quelconque des revendications précédentes, dans lequel un critère d'exclusion comprend des informations d'uniformité de blastomère à t2, des informations de multinucléarité au stade de deux blastomères et/ou au stade de quatre blastomères, et/ou des informations de clivage d'un blastomère directement à trois blastomères.

9. Procédé selon l'une quelconque des revendications précédentes,
dans lequel un critère d'exclusion est que t2 est supérieur à 31,8 heures ; et/ou
dans lequel un critère d'exclusion est que t5 est inférieur à 49 heures ; et/ou
dans lequel un critère d'exclusion est que cc2b est supérieur à 13,1 heures ; et/ou
dans lequel un critère d'exclusion est que cc3 est supérieur à 17,6 heures ; et/ou
dans lequel un critère d'exclusion est que s2 est supérieur à 2,1 heures ; et/ou
dans lequel un critère d'exclusion est que s3 est supérieur à 13,8 heures,
dans lequel cc2b est t4-t2 ; t4 est un temps de clivage pour un embryon à 4 blastomères ; S2 est t4-t3 ; et S3 est t8-t5 ; et t8 est un temps de clivage pour un embryon à 8 blastomères.

10. Procédé selon l'une quelconque des revendications précédentes, dans lequel l'embryon humain est surveillé dans un incubateur.

11. Procédé selon l'une quelconque des revendications précédentes, dans lequel l'embryon humain est surveillé par acquisition d'image telle qu'une acquisition d'image au moins une fois par heure, de préférence une acquisition d'image au moins une fois par demi-heure.

**Fig. 1**

## Fig. 2

## Fig. 3

**Fig. 4**

t2

**Fig. 5**

t3=t2

**Fig. 6a**

**Fig. 6b**

**Fig. 7**

**Fig. 8**

**Fig. 9**

# Fig. 10

**t2**

p=0.043

**t3**

p=0.007

**t5**

p=0.001

# Fig. 11

**Fig. 12**

**Fig. 13**

**Fig. 14**

**Fig. 15**

**Fig. 16**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011025736 A
- EP 2282210 A
- WO 2007144001 PCT
- WO 2007042044 A
- WO 2004056265 PCT
- WO 2004056265 A

**Non-patent literature cited in the description**

- **M. MESEGUER et al.** The Use of Morphokinetics as a Predictor of Embryo Implantation. *Human Reproduction,* (10), ISSN 0268-1161, 2658-2671
- **ALIKANI, M. ; CALDERON, G. ; TOMKIN, G. ; GARRISI, J. ; KOKOT, M. ; COHEN, J.** Cleavage anomalies in early human embryos and survival after prolonged culture in-vitro. *Hum Reprod,* 2000, vol. 15 (12), 2634-2643
- **ARAV, A. ; AROYO, A. ; YAVIN, S ; ROTH, Z.** Prediction of embryonic developmental competence by time-lapse observation and 'shortest-half' analysis. *Reprod Biomed Online,* 2008, vol. 17 (5), 669-675
- **BOS-MIKICH, A. ; MATTOS, A.L.G. ; FERRARI, A.N.** Early cleavage of human embryos: an effective method for predicting successful IVF/ICSI outcome. *Hum Reprod,* 2001, vol. 16 (12), 2658-2661
- **BREZINOVA, J. ; OBORNA, I. ; SVOBODOVA, M ; FINGEROVA, H.** Evaluation of day one embryo quality and IVF outcome - a comparison of two scoring systems. *Reprod Biol Endocrinol,* 2009, vol. 7 (9
- **CIRAY HN ; KARAGENC L ; ULUG U ; BENER F ; BAHCECI M.** Use of both early cleavage and day 2 mononucleation to predict embryos with high implantation potential in intracytoplasmic sperm injection cycles. *Fertil Steril,* 2005, vol. 84, 1411-6
- **CIRAY HN ; KARAGENC L ; ULUG U ; BENER F ; BAHCECI M.** Early cleavage morphology affects the quality and implantation potential of day 3 embryos. *Fertil Steril,* 2006, vol. 85, 358-65
- **CRUZ, M. ; GADEA, B. ; GARRIDO, N. ; PEDERSEN, K.S. ; MARTINEZ, M. ; PEREZ-CANO, I. ; MUÑOZ, M ; MESEGUER, M.** Embryo quality, blastocyst and ongoing pregnancy rates in oocyte donation patients whose embryos were monitored by time-lapse imaging. *J Assist Reprod Genet,* 2011
- **CRUZ, M. ; GARRIDO, N. ; PEREZ-CANO, I. ; MUÑOZ, M. ; PELLICER, A. ; MARTINEZ, M. ; GADEA, B. ; SELLÉS, E. ; BETERSEN, J ; MESEGUER, M.** Time lapse video analysis provide precise division kinetics of cultured embryos which corellates with blastocyst formation and quality. *Abstracts of the 26th Annual Meeting of ESHRE,* 2010, i203
- **CRUZ, M. ; NICOLÁS, G. ; INMACULADA, P.-C. ; R. NIELS ; M. MANUEL ; M. MARCOS.** Comparative study of embryo quality, blastocyst and ongoing pregnancy rates in oocyte donation patients sharing embryoscope and standard incubator. *Fertil Steril,* 2010, vol. 94 (4), S78
- **DE LOS SANTOS, M. J. ; ESCRICH, L. ; GRAU, N. ; PELLICER, A. ; ROMERO, J.L. ; ESCRIBÁ, M.J.** Development of tripronucleated ICSI-derived embryos using real time assessment. *Abstracts of the 26th Annual Meeting of ESHRE,* 2010, i192
- **ELLIOTT, T. ; KAHN, J. ; LOWDERMAN, J. ; WRIGHT, G. ; CHANG, C. ; BERNAL, D. ; KORT, H ; NAGY, Z.** Time-lapse video evidence of murine embryonic developmental regression in sub-optimal culture conditions using Embryoscope™. *Abstracts of the 26th Annual Meeting of ESHRE,* 2010, i192
- **ESCRICH, L. ; GRAU, N. ; GARCIA-BAUTISTA, A. ; GALÁN, A. ; PELLICER, A ; ESCRIBÁ, M.J.** Real-time evaluation of the different stages of meiosis during spontaneous in vitro maturation of human GV oocytes. *Fertil Steril,* 2010, vol. 94 (4), S141
- **FANCSOVITS, P. ; TAKÁCS, F. Z. ; TÓTHNÉ, G. Z. ; PAPP, Z ; URBANCSEK, J.** Examination of Early Cleavage and its Importance in IVF Treatment. *J Reproduktionsmed Endokrinol,* 2006, vol. 3 (6), 367-372
- **FAWCETT, T.** An introduction to ROC analysis. *Pattern Recognition Letters,* 2006, vol. 27, 861-874

- **FENWICK, J. ; PLATTEAU, P. ; MURDOCH, A.P. ; HERBERT, M.** Time from insemination to first cleavage predicts developmental competence of human preimplantation embryos in vitro. *Hum Reprod,* 2002, vol. 17 (2), 407-412
- **FINN, A. ; SCOTT, L. ; O'LEARY, T. ; DAVIES, D ; HILL, J.** Sequential embryo scoring as a predictor of aneuploidy in poor-prognosis patients. *Reprod Biomed Online,* 2010, vol. 21, 381-390
- **GIORGETTI, C. ; HANS, E. ; TERRIOU, P. ; SALZMANN, J. ; BARRY, B. ; CHABERT-ORSINI, V. ; CHINCHOLE, J.M. ; FRANQUEBALME, J.P. ; GLOWACZOWER, E. ; SITRI, M.C.** Early cleavage: an additional predictor of high implantation rate following elective single embryo transfer. *Reproductive biomedicine online,* 2007, vol. 14 (1), 85-91
- **GRAU, N. ; ESCRICH, L. ; RAMSING, N. ; GARCIA-BAUTISTA, A. ; PELLICER, A ; ESCRIBÁ, M.J.** A new approach to determining the optimal duration of MII arrest in vitro-matured human GV oocytes. *Fertil Steril,* 2010, vol. 94 (4), S141
- **GRISART, B. ; MASSIP, A ; DESSY, F.** Cinematographic analysis of bovine embryo development in serum-free oviduct-conditioned medium. *J Reprod Fertil,* 1994, vol. 101, 257-264
- **HARDARSON, T. ; HANSON, C. ; SJÖGREN, A ; LUNDIN, K.** Human embryos with unevenly sized blastomeres have lower pregnancy and implantation rates: indications for aneuploidy and multinucleation. *Hum Reprod,* 2001, vol. 16 (2), 313-318
- **HARDARSON, T. ; LÖFMAN, C. ; COULL, G. ; SJÖGREN, A. ; HAMBERGER, L ; EDWARDS, R.G.** Internalization of cellular fragments in a human embryo: time-lapse recordings. *Reprod Biomed Online,* 2002, vol. 5 (1), 36-38
- **HERRERO, J. ; ALBERTO, T. ; RAMSING, N. B. ; DE LOS SANTOS, M. J. ; ESCRICH, L ; MESEGUER, M.** Linking successful implantation with the exact timing of cell division events obtained by time-lapse system in the embryoscope. *Fertil Steril,* 2010, vol. 94 (4), S149
- **HESTERS, L. ; PRISANT, N. ; FANCHIN, R. ; MÉNDEZ LOZANO, D. H. ; FEYEREISEN, E. ; FRYDMAN, R. ; TACHDJIAN, G ; FRYDMAN, N.** Impact of early cleaved zygote morphology on embryo development and in vitro fertilization-embryo transfer outcome: a prospective study. *Fertil Steril,* 2008, vol. 89 (6), 1677-1684
- **HOLM, P ; SHUKRI, N.N. ; VAJTA, G. ; BOOTH, P. ; BENDIXEN, C ; CALLESEN, H.** Developmental kinetics of the first cell cycles of bovine in vitro produced embryos in relation to their in vitro viability and sex. *Theriogenology,* 1998, vol. 50, 1285-1299
- **HOLM, P. ; BOOTH, P.J ; CALLESEN, H.** Kinetics of early in vitro development of bovine in vivo- and in vitro-derived zygotes produced and/or cultured in chemically defined or serum-containing media. *Reprod,* 2002, vol. 123, 553-565
- **HOLM, P. ; BOOTH, P.J. ; CALLESEN, H.** Developmental Kinetics of Bovine Nuclear Transfer and Parthenogenetic Embryos. *Cloning and stem cells,* 2003, vol. 5 (2), 133-142
- **HOLTE, J. ; BERGLUND, L. ; MILTON, K. ; GARELLO, C. ; GENNARELLI, G. ; REVELLI, A ; BERGH, T.** Construction of an evidence-based integrated morphology cleavage embryo score for implantation potential of embryos scored and transferred on day 2 after oocyte retrieval. *Hum Reprod,* 2007, vol. 22 (2), 548-557
- **JONES, G.M. ; CRAM, D.S. ; SONG, B. ; KOKKALI, G. ; PANTOS, K ; TROUNSON, A.O.** Novel strategy with potential to identify developmentally competent IVF blastocysts. *Human reproduction (Oxford, England),* 2008, vol. 23 (8), 1748-1759
- **LECHNIAK, D. ; PERS-KAMCZYC, E. ; PAWLAK, P.** Timing of the first zygotic cleavage as a marker of developmental potential of mammalian embryos. *Reprod Biol,* 2008, vol. 8 (1), 23-42
- **LEMMEN, J.G. ; AGERHOLM, I ; ZIEBE, S.** Kinetic markers of human embryo quality using time-lapse recordings of IVF/ICSI-fertilized oocytes. *Reprod Biomed Online,* 2008, vol. 17 (3), 385-391
- **LEQUARRE, A.S. ; MARCHANDISE, J. ; MOREAU, B. ; MASSIP, A ; DONNAY, I.** Cell Cycle Duration at the Time of Maternal Zygotic Transition for In vitro Produced Bovine Embryos: Effect of Oxygen Tension and Transcription Inhibition. *Biol Reprod,* 2003, vol. 69, 1707-1713
- **LOPES, A.S. ; GREVE, T ; CALLESEN, H.** Quantification of embryo quality by respirometry. *Theriogenology,* 2007, vol. 67, 21-31
- **LOPES, A.S. ; LANE, M ; THOMPSON, J.G.** Oxygen consumption and ROS production are increased at the time of fertilization and cell cleavage in bovine zygotes. *Hum Reprod,* 2010, vol. 25 (11), 2762-2773
- **LOPES, A.S. ; LARSEN, L.H. ; RAMSING, N. ; LOVENDAHL, P. ; RATY, M. ; PEIPPO, J. ; GREVE, T ; CALLESEN, H.** Respiration rates of individual bovine in vitro-produced embryos measured with a novel, non-invasive and highly sensitive microsensor system. *Reprod,* 2005, vol. 130, 669-679
- **LOPES, A.S. ; MADSEN, S.E. ; RAMSING, N.B. ; LOVENDAHL, P. ; GREVE, T ; CALLESEN, H.** Investigation of respiration of individual bovine embryos produced in vivo and in vitro and correlation with viability following transfer. *Hum Reprod,* 2007, vol. 22, 558-566
- **LOPES, A.S. ; WRENZYCKI, C. ; RAMSING, N.B. ; HERRMANN, D. ; NIEMANN, H. ; LOVENDAHL, P. ; GREVE, T ; CALLESEN, H.** Respiration rates correlate with mRNA expression of G6PD and GLUT1 genes in individual bovine in vitro-produced blastocysts. *Theriogenology,* 2007, vol. 68, 223-236

- **LUNDIN, K ; BERGH, C ; HARDARSON, T.** Early embryo cleavage is a strong indicator of embryo quality in human IVF. *Hum Reprod (Oxford, England),* 2001, vol. 16 (12), 2652-2657
- **MAGLI, M.C. ; GIANAROLI,L. ; FERRARETTI, A.P. ; LAPPI, M. ; RUBERTI, A ; FARFALLI,V.** Embryo morphology and development are dependent on the chromosomal complement. *Fertil Steril,* 2007, vol. 87 (3), 534-541
- **MAJERUS, V. ; LEQUARREÂ, A.S. ; FERGUSON, E.M. ; KAIDI, S. ; MASSIP, A. ; DESSY, F ; DONNAY, I.** Characterization of Embryos Derived From Calf Oocytes: Kinetics of Cleavage, Cell Allocation to Inner Cell Mass, and Trophectoderm and Lipid Metabolism. *Mol Reprod Dev,* 2000, vol. 57, 346-352
- **MASTENBROEK, S. ; TWISK, M. ; VAN ECHTEN-ARENDS, J. ; SIKKEMA-RADDATZ, B. ; KOREVAAR, J.C. ; VERHOEVE, H.R. ; VOGEL, N.E. ; ARTS, E.G. ; DE VRIES, J.W. ; BOSSUYT, P.M.** In vitro fertilization with preimplantation genetic screening. *The New England journal of medicine,* 2007, vol. 357 (1), 9-17
- **MENG, L. ; JASTROMB, N. ; ALWORTH, S ; JAIN, J.** Remote Monitoring and Evaluation of Early Human Embryo Developmentby a Robotic-Operated Culture-Imaging System. *Fertil Steril,* 2009, S7
- **MESEGUER, M. ; HERRERO, J. ; TEJERA, A. ; DE LOS SANTOS, M. J. ; ESCRICH, L. ; GARRIDO, N ; RAMSING, N.** Embryos with too early first cleavage fail to implant; correlation between exact timing of first cleavage and successful implantation. *Abstracts of the 26th Annual Meeting of ESHRE,* 2010, i58-i59
- **MESEGUER, M. ; HILLIGSØE, K.M. ; PEDERSEN, K.S. ; HERRERO, J. ; TEJERA, A ; GARRIDO, N.** Pregnancy rates after incubation in new time-lapse incubator (EmbryoScope) providing detailed information about embryo development compared to incubation in a standard incubator. *Fertil Steril,* 2010, vol. 94 (4), S150
- **MIO Y.** Morphological analysis of human em- bryonic development using time-lapse cinematography. *J Mamm Ova Res,* 2006, vol. 23, 27-36
- **MIO, Y ; MAEDA, K.** Time-lapse cinematography of dynamic changes occurring during in vitro development of human embryos. *Am J Obstet Gynecol,* 2008, vol. 199 (6), 660.e1-e5
- **MUÑOZ, M. ; MESEGUER, M. ; CRUZ, M. ; PÉREZ-CANO, I. ; PELLICER, A. ; GADEA, B. ; MARTINEZ, M. ; FORTUÑO, S. ; GUNDERSEN, J ; GARRIDO, N.** Morphometric variations and differences in embryo cleavage kinetics associated with fertilization procedure: classic IVF vs. ICSI. *Abstracts of the 26th Annual Meeting of ESHRE,* 2010, i202-i203
- **NAKAHARA, T. ; IWASE, A. ; GOTO, M. ; HARATA, T. ; SUZUKI, M. ; LENAGA, M. ; KOBAYASHI, H. ; TAKIKAWA, S. ; MANABE, S. ; KIKKAWA, F.** Evaluation of the safety of time-lapse observations for human embryos. *J Assist Reprod Genet,* 2010, vol. 27, 93-96
- **NEUBER, E. ; RINAUDO, P. ; TRIMARCHI, J.R. ; SAKKAS, D.** Sequential assessment of individually cultured human embryos as an indicator of subsequent good quality blastocyst development. *Hum Reprod,* 2003, vol. 18 (6), 1307-1312
- **OH, S.J. ; GONG, S.P. ; LEE, S.T. ; LEE, E.J. ; LIM, J.M.** Light intensity and wavelength during embryo manipulation are important factors for maintaining viability of preimplantation embryos in vitro. *Fertil Steril,* 2007, vol. 88 (2), 1150-1157
- **OTTOSEN, L.D. ; HINDKJAER, J. ; INGERSLEV, J.** Light exposure of the ovum and preimplantation embryo during ART procedures. *J Assist Reprod Genet,* 2007, vol. 24, 99-103
- **PATERNOT, G. ; DEVROE, J. ; DEBROCK, S. ; D'HOOGHE, T.M. ; SPIESSENS, C.** Intra- and inter-observer analysis in the morphological assessment of early-stage embryos. *Reprod Biol Endocrinol,* 2009, vol. 7 (105
- **PAYNE, D. ; FLAHERTY, S.P. ; BARRY, M.F ; MATTHEWS, C.D.** Preliminary observations on polar body extrusion and pronuclear formation in human oocytes using time-lapse video cinematography. *Hum Reprod,* 1997, vol. 12 (3), 532-541
- **PETERSEN, C. G. ; MAURI, A. L. ; FERREIRA, R. ; BARUFFI, R. L. R. ; FRANCO, JR, J. G.** Embryo Selection by the First Cleavage Parameter Between 25 and 27 Hours After ICSI. *J Assist Reprod Genet,* 2001, vol. 18 (4), 209-212
- **PRIBENSZKY, C. ; LOSONCZI, E. ; MOLNÁR, M. ; LANG, Z. ; MÁTYÁS, S. ; RAJCZY, K. ; MOLNÁR, K. ; KOVÁCS, P., NAGY, P. ; CONCEICAO, J ; VAJTA, G.** Prediction of in-vitro developmental competence of early cleavage-stage mouse embryos with compact time-lapse equipment. *Reprod Biomed Online,* 2010, vol. 20, 371-379
- **RACOWSKY, C. ; OHNO-MACHADO, L. ; KIM, J ; BIGGERS, J.D.** Is there an advantage in scoring early embryos on more than one day?. *Hum Reprod,* 2009, vol. 24 (9), 2104-2113
- **RAMSING, N. B. ; CALLESEN, H.** Detecting timing and duration of cell divisions by automatic image analysis may improve selection of viable embryos. *Fertil Steril,* 2006, vol. 86 (3), S189
- **RAMSING, N. B. ; BERNTSEN, J ; CALLESEN, H.** Automated detection of cell division and movement in time-lapse images of developing bovine embryos can improve selection of viable embryos. *Fertil Steril,* 2007, vol. 88 (1), S38

- **REED, M.L. ; HAMIC, A. ; THOMPSON, D.J. ; CA-PERTON, C.L.** Continuous uninterrupted single medium culture without medium renewal versus sequential media culture: a sibling embryo study. *Fertil Steril,* 2009, vol. 92, 1783-1786
- **REICHMAN, D.E. ; JACKSON, K.V. ; RACOWSKY, C.** Incidence and development of zygotes exhibiting abnormal pronuclear disposition after identification of two pronuclei at the fertilization check. *Fertil Steril,* 2010, vol. 94 (3), 965-970
- **SAKKAS, D. ; PERCIVAL, G. ; D'ARCY, Y. ; SHAR-IF, K ; AFNAN, M.** Assessment of early cleaving in vitro fertilized human embryos at the 2-cell stage before transfer improves embryo selection. *Fertil Steril,* 2001, vol. 76 (6), 1150-1156
- **SAKKAS, D. ; SHOUKIR, Y. ; CHARDONNENS, D. ; GRACE BIANCHI, P ; CAMPANA, A.** Early cleavage of human embryos to the two-cell stage after intracytoplasmic sperm injection as an indicator of embryo viability. *Hum Reprod,* 1998, vol. 13, 182-187
- **SCOTT, L.** Pronuclear scoring as a predictor of embryo development. *Reproductive biomedicine online,* 2003, vol. 6 (2), 201-214
- **SCOTT, L ; RAMSING, N.B.** Oocyte and embryo respiration rate measurements in a clinical setting - Potential to improve embryo selection?. *The Clinical Embryologist,* 2007, vol. 10 (2), 5-9
- **SCOTT, L. ; BERNTSEN, J. ; DAVIES, D. ; GUNDERSEN, J. ; HILL, J ; RAMSING, N.** Symposium: Innovative techniques in human embryo viability assessment Human oocyte respiration-rate measurement - potential to improve oocyte and embryo selection?. *Reprod BioMed Online,* 2008, vol. 17 (4), 461-469
- **SCOTT, L. ; FINN, A. ; KLOOS, B ; DAVIES, D.** The origin and consequences of Day-2 multinucleation of human Embryos. *Abstracts of the 26th Annual Meeting of ESHRE,* 2010, i195
- **SCOTT, L. ; FINN, A. ; O'LEARY, T. ; MCLELLAN, S ; HILL, J.** Morphologic parameters of early cleavage-stage embryos that correlate with fetal development and delivery: prospective and applied data for increased pregnancy rates. *Hum Reprod,* 2007, vol. 22 (1), 230-240
- **SELI, E. ; VERGOUW, C.G. ; MORITA, H. ; BOTROS, L. ; ROOS, P. ; LAMBALK, C.B. ; YAMASHITA, N. ; KATO, O ; SAKKAS, D.** Noninvasive metabolomic profiling as an adjunct to morphology for noninvasive embryo assessment in women undergoing single embryo transfer. *Fertility and sterility,* 2010, vol. 94 (2), 535-542
- **SHOUKIR, Y. ; CAMPANA, A. ; FARLEY, T. ; SAKKAS, D.** Early cleavage of in-vitro fertilized human embryos to the 2-cell stage: a novel indicator of embryo quality and viability. *Hum Reprod,* 1997, vol. 12, 1531-1536
- **SIFER, C. ; HANDELSMAN, D. ; GRANGE, E. ; PORCHER, R. ; PONCELET, C. ; MARTIN-PONT, B. ; BENZACKEN, B ; WOLF, J.P.** An auto-controlled prospective comparison of two embryos culture media (G III series versus ISM) for IVF and ICSI treatments. *Journal of assisted reproduction and genetics,* 2009, vol. 26, 575-581
- **SOMFAI, T. ; INABA, Y. ; AIKAWA, Y. ; OHTAKE, M. ; KOBAYASHI, S. ; KONISHI, K ; IMAI, K.** Relationship between the length of cell cycles, cleavage pattern and developmental competence in bovine embryos generated by in vitro fertilization or parthenogenesis. *J Reprod Dev,* 2010, vol. 56 (2), 200-207
- **TAKENAKA, M. ; HORIUCHI, T ; YANAGIMACHI, R.** Effects of light on development of mammalian zygotes. *PNAS,* 2007, vol. 104 (36), 14289-14293
- **TEJERA, A. ; HERRERO, J. ; RAMSING, N. B. ; GARRIDO, N. ; GRAU, N ; MESEGUER, M.** Embryo respiration measurements used for quantification of embryo quality; embryo respiration correlates with ongoing pregnancy and implantation in oocyte donation program. *Fertil Steril,* 2010, vol. 94 (4), S67-S68
- **TEJERA, A. ; HERRERO, J. ; RAMSING, N. ; PELLICER, A. ; GARRIDO, N. ; GRAU, N. ; DE LOS SANTOS, M.J. ; MESEGUER, M.** Quantification of oocyte quality through respiration patterns correlates oxygen consumption with fertilization and subsequent implantation. *Hum Reprod,* 2010, vol. 25 (1
- **TERRIOU, P. ; GIORGETTI, C. ; HANS, E. ; SALZMANN, J. ; CHARLES, O. ; CIGNETTI, L. ; AVON, C ; ROULIER, R.** Relationship between even early cleavage and day 2 embryo score and assessment of their predictive value for pregnancy. *Reproductive biomedicine online,* 2007, vol. 14 (3), 294-299
- **TURCZYNSKI, C.J. ; CHANG, C. ; HOVIS, W. ; MARINO, A ; LONDON, S.L.** Culture of Embryos in an Environment Shielded from Exposure to Electro-Magnetic Fields has no Effect on IVF Outcome. *Fertil Steril,* 1999, vol. 72 (3), 1
- **UGAJIN, T. ; TERADA, Y. ; HASEGAWA, H. ; VELAYO, C.L. ; NABESHIMA, H. ; YAEGASHI, N.** Aberrant behavior of mouse embryo development after blastomere biopsy as observed through time-lapse cinematography. *Fertil Steril,* 2010, vol. 93 (8), 2723-2728
- **VAJTA, G. ; KORÖSI, T. ; DU, Y. ; NAKATA, K. ; LEDA, S. ; KUWAYAMA, M. ; NAGY, Z.P.** The Well-of-the-Well system: an efficient approach to improve embryo development. *Reprod Biomed Online,* 2008, vol. 17 (1), 73-81

- **VAN MONTFOORT, A.P.A. ; DUMOULIN, J. C.M. ; KESTER, A.D.M. ; EVERS, J.L.H.** Early cleavage is a valuable addition to existing embryo selection parameters: a study using single embryo transfers*. *Hum Reprod,* 2004, vol. 19 (9), 2103-2108
- **VERMILYEA, M.D. ; GRAHAM, J.R. ; RICHTER, K.S. ; MOTTLA, G.L. ; TUCKER, M.J.** Redefining the mouse embryo assay (mea): novel time-lapse imagery and continuous surveillance vs. 'Snap shot' observation. *Fertil Steril,* 2010, vol. 94 (4), S212
- **WALE, P.L. ; GARDNER, D.K.** Time-lapse analysis of mouse embryo development in oxygen Gradients. *Reprod BioMed Online,* 2010, vol. 21 (3), 402-410
- **WEITZMAN, V.N. ; SCHNEE-RIESZ, J. ; BENADIVA, C. ; NULSEN, J. ; SIANO, L. ; MAIER, D.** Predictive value of embryo grading for embryos with known outcomes. *Fertil Steril,* 2010, vol. 93 (2), 658-662
- **WINDT, M.-L. ; KRUGER, T.F. ; COETZEE, K. ; LOMBARD, C.J.** Comparative analysis of pregnancy rates after the transfer of early dividing embryos versus slower dividing embryos. *Hum Reprod,* 2004, vol. 19 (5), 1155-1162
- **WONG, C.C. ; LOEWKE, K.E. ; BOSSERT, N.L. ; BEHR, B. ; DE JONGE, C.J. ; BAER, T.M. ; REIJO PERA, R.A.** Non-invasive imaging of human embryos before embryonic genome activation predicts development to the blastocyst stage. *Nat Biotechnol,* 2010, vol. 28 (10), 1115-1121
- **YAKIN, K. ; BALABAN, B. ; URMAN, B.** Impact of the presence of one or more multinucleated blastomeres on the developmental potential of the embryo to the blastocyst stage. *Fertil Steril,* 2005, vol. 83 (1), 243-245
- **YAMAGATA, K. ; SUETSUGU, R. ; WAKAYAMA, T.** Long-term, six-dimensional live-cell imaging for the mouse preimplantation embryo that does not affect full-term development. *J Reprod Dev,* 2009, vol. 55, 328-331
- **YAMAZAKI, T. ; YAMAGATA, K. ; BABA, T.** Time-lapse and retrospective analysis of DNA methylation in mouse preimplantation embryos by live cell imaging. *Dev Biol,* 2007, vol. 304, 409-419
- **YANG, W.J. ; HWU, Y.M. ; LEE, R.K. ; LI, S.H. ; FLEMING, S.** Early-cleavage is a reliable predictor for embryo implantation in the GnRH agonist protocols but not in the GnRH antagonist protocols. *Reprod Biol Endocrinol,* 2009, vol. 7 (20
- **ZHANG, J.Q. ; LI, X.L. ; PENG, Y. ; GUO, X ; HENG, B.C. ; TONG, G.Q.** Reduction in exposure of human embryos outside the incubator enhances embryo quality and blastulation rate. *Reprod Biomed Online,* 2010, vol. 20, 510-515